(19) Europäisches Patentamt / European Patent Office / Office européen des brevets

(11) **EP 3 958 738 B1**

(12) **FASCICULE DE BREVET EUROPEEN**

(45) Date de publication et mention de la délivrance du brevet:
**14.06.2023 Bulletin 2023/24**

(21) Numéro de dépôt: **20731910.4**

(22) Date de dépôt: **25.04.2020**

(51) Classification Internationale des Brevets (IPC):
*A61B 5/11* *(2006.01)*    *A61B 5/103* *(2006.01)*
*G16H 50/20* *(2018.01)*   *G16H 20/30* *(2018.01)*
*G16H 40/67* *(2018.01)*   *A61B 5/00* *(2006.01)*

(52) Classification Coopérative des Brevets (CPC):
**A61B 5/6807; A61B 5/1121; G16H 20/30;
G16H 40/67; G16H 50/20;** A61B 2560/0214

(86) Numéro de dépôt international:
**PCT/FR2020/050714**

(87) Numéro de publication internationale:
**WO 2020/217037 (29.10.2020 Gazette 2020/44)**

(54) **PROCEDE ET SYSTEME DE DETERMINATION D'UNE VALEUR DE PARAMETRE BIOMECANIQUE AVANCE DE DEMARCHE**

VERFAHREN UND SYSTEM ZUR BESTIMMUNG EINES WERTES EINES FORTGESCHRITTENEN BIOMECHANISCHEN GANGPARAMETERS

METHOD AND SYSTEM FOR DETERMINING A VALUE OF AN ADVANCED BIOMECHANICAL GAIT PARAMETER

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **25.04.2019 FR 1904412**

(43) Date de publication de la demande:
**02.03.2022 Bulletin 2022/09**

(73) Titulaires:
• **Zhor Tech**
  **54000 Nancy (FR)**
• **Digitsole**
  **54000 Nancy (FR)**

(72) Inventeur: **OUMNIA, Karim**
  **54000 Nancy (FR)**

(74) Mandataire: **A.P.I. Conseil
Immeuble Newton
4, rue Jules Ferry
64000 Pau (FR)**

(56) Documents cités:
   **EP-A1- 3 038 042      EP-A1- 3 257 437
   CN-A- 105 631 195     US-A1- 2008 214 360**

• **RAMPP ALEXANDER ET AL: "Inertial Sensor-Based Stride Parameter Calculation From Gait Sequences in Geriatric Patients", IEEE TRANSACTIONS ON BIOMEDICAL ENGINEERING, IEEE SERVICE CENTER, PISCATAWAY, NJ, USA, vol. 62, no. 4, 1 avril 2015 (2015-04-01), pages 1089-1097, XP011575709, ISSN: 0018-9294, DOI: 10.1109/TBME.2014.2368211 [extrait le 2015-03-17]**

## Description

L'invention s'intéresse au domaine de la caractérisation de la démarche et plus particulièrement à la détermination de valeurs de paramètres biomécaniques de démarche, pouvant trouver une application dans le suivi d'activités quotidiennes ou sportives, ou encore le suivi de l'état physiologique du sujet d'étude. L'invention concerne un procédé de détermination d'une valeur de paramètre biomécanique avancé de démarche à partir de données générées par un ou plusieurs capteurs de mouvement d'une semelle. L'invention concerne en outre un système de détermination d'une valeur de paramètre biomécanique avancé de démarche pouvant mettre en oeuvre un tel procédé.

### [Art antérieur]

La démarche humaine est un processus complexe et cyclique visant principalement à soutenir la position verticale et à maintenir l'équilibre dans des conditions statiques et dynamiques et nécessitant pour cela la synergie des muscles, des os et du système nerveux.

De nombreux systèmes ont été développés au fil des années pour étudier la démarche humaine. Ces systèmes comportent par exemple l'utilisation de chaussures équipées de capteurs de pression ou de capteurs inertiels positionnés dans la semelle, sur la chaussure ou encore au niveau de la cheville. En particulier, plusieurs équipes se sont concentrées sur l'utilisation et le traitement des données de capteurs inertiels pour l'analyse du mouvement humain. Ces systèmes, basés sur un ou plusieurs capteurs, fournissent aujourd'hui des valeurs de paramètres « marche » classiques indiquant par exemple des informations sur le nombre de pas grâce à des calculs réalisés le plus souvent intégralement sur des terminaux externes.

La marche humaine peut refléter l'état de certains systèmes sensoriels, neuronaux et biomécaniques impliqués dans la démarche. Ainsi, d'autres équipes ont cherché à étudier les paramètres biomécaniques avancés de la démarche tels que ceux dépendant de la relation spatiale des deux pieds, par exemple la longueur et la largeur du pas. La mesure de tels paramètres biomécaniques de la démarche nécessite généralement un équipement spécialisé tel que des systèmes vidéo de capture de mouvement, limitant ainsi les mesures au laboratoire. Les progrès technologiques dans les unités de mesure inertielles miniatures (accéléromètres et gyroscopes) offrent la possibilité de mesurer les progrès réalisés à l'extérieur du laboratoire.

Cependant, l'estimation des paramètres biomécaniques avancés de la démarche à partir de tels capteurs entraine généralement des écart-types trop élevés pour pouvoir être exploités correctement. Elles nécessitent alors la mise en place dans les algorithmes de traitement de nombreuses corrections de dérives.

La demande EP3038042 A1 divulgue une méthode de recommandation de chaussure de sport à un utilisateur s'appuyant sur l'analyse de données de capteurs afin de reconnaître notamment l'activité pratiquée par l'utilisateur et des moments clés de sa démarche.

Par exemple, l'analyse de paramètres biomécaniques avancés de la démarche a été appliqué dans un contexte de vieillissement, risque de chute, lésions de la moelle épinière, neuropathie diabétique et affections neurologiques (Rebula et al. Gait Posture. 2013 September ; 38(4): 974-980). Cette étude a montré que l'utilisation de données de mouvement de type accélération et vitesse angulaire nécessitait la mise en place de corrections basées sur des hypothèses de linéarité de l'erreur et induisant alors des approximations sur les valeurs de paramètres calculés.

L'élévation du pied pendant la phase de foulée est un indicateur important de la qualité et de la sécurité de la marche chez les sujets handicapés. Dans une étude dédiée à l'étude de ce paramètre, il a été obtenu une erreur moyenne d'environ 4,1 centimètres (cm) (Mariani et al. IEEE Trans. Bio-Med. Eng. 2012;59:3162-3168). Tandis que dans une autre étude, d'après les auteurs, l'observation d'une dégradation des performances était principalement due à l'algorithme de détection de phase pied plat, qui présentait certaines limites lorsque la démarche n'était pas très naturelle, notamment dans le cas d'une démarche relative à un individu devant franchir des obstacles (Benoussaad et al. Sensors (Basel). 2015 Dec 23;16(1). pii: E12.). Ainsi, les systèmes actuels de détermination de valeurs de paramètres biomécaniques de la démarche ne sont pas satisfaisants, puisque ces derniers ne permettent pas de calculer avec précision de tels paramètres.

### [Problème technique]

L'invention a pour but de remédier aux inconvénients de l'art antérieur. En particulier, l'invention a pour but de proposer un procédé de détermination d'une valeur de paramètre biomécanique avancé de démarche et capable de générer des valeurs de paramètre biomécanique avancé de démarche présentant une variabilité plus faible. En outre, l'absence de besoin fort en ressources de calcul pour les étapes du procédé rend possible sa mise en oeuvre en temps réel.

L'invention a en outre pour but de proposer un système de détermination d'une valeur de paramètre biomécanique avancé de démarche, ledit système étant capable de générer des valeurs de paramètres biomécaniques avancés de démarche présentant une variabilité plus faible.

### [Brève description de l'invention]

A cet effet, l'invention porte sur un procédé de détermination d'une valeur de paramètre biomécanique avancé de démarche à partir de données générées par un ou plusieurs capteurs de mouvement d'une semelle, ledit procédé, exécuté par un ou plusieurs modules d'analyse, comportant :

- Une étape d'acquisition de données générées par un ou plusieurs capteurs de mouvement d'une semelle, lesdites données comportant des valeurs d'accélération, de vitesse angulaire et/ou de champs magnétique ;

- Une étape de calcul d'une valeur d'orientation de la semelle, ladite valeur d'orientation de la semelle étant calculée par rapport à un référentiel terrestre à partir de données comportant les valeurs d'accélération, de vitesse angulaire et/ou de champs magnétique ;

- Une étape d'identification d'une activité associée à une unité de mouvement, ladite identification d'une activité comportant la comparaison de valeurs de référence d'activité à des valeurs sélectionnées parmi : les valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle ;

- Une étape d'identification d'au moins deux moments clés de l'unité de mouvement pour laquelle une activité a été identifiée, ladite étape d'identification de moment clés comportant la comparaison de valeurs de paramètres de mouvement à au moins un seuil prédéterminé, ledit seuil prédéterminé étant associé à l'activité identifiée et lesdites valeurs de paramètres de mouvement comportant les valeurs d'accélération, de vitesse angulaire et/ou d'orientation de la semelle ; et

- Une étape de détermination d'une valeur de paramètre biomécanique avancé de marche, ladite détermination comportant le calcul d'une valeur de paramètre biomécanique de démarche pour au moins un des moments clés identifiés et à partir des valeurs d'accélération, de vitesse angulaire et/ou d'orientation de la semelle.

Un tel procédé se distingue notamment de l'existant par la présence d'une identification d'une activité associée à une unité de mouvement d'une part et d'une détermination d'une valeur de paramètre biomécanique de marche pour au moins un moment clés identifié d'autre part. Alors que les systèmes classiques cherchent à identifier des paramètres de démarche souvent à partir d'une séquence éventuellement prétraitée, un procédé selon l'invention se base sur une première identification d'une activité puis sur la détermination de valeurs de paramètres seulement sur les unités de mouvement ayant été associées à une activité et sur la base de seuils adaptés à ladite activité. Ainsi, la précision du calcul est améliorée et la variabilité des résultats en est diminuée. Un procédé selon l'invention permet alors de suivre la démarche d'un utilisateur de façon fiable. En outre, certains paramètres biomécaniques n'auront de sens que s'ils sont associés à un moment clés d'un cycle. Or, grâce à ces étapes, le procédé permet pour la première fois d'obtenir avec justesse des valeurs de paramètres biomécanique associés à des moments précis devenant alors des paramètres biomécaniques avancés. Contrairement à la littérature où les paramètres sont calculés pour tous les temps, ici le calcul se fait de préférence pour un moment particulier identifié.

**Selon d'autres caractéristiques optionnelles du procédé :**

- le paramètre biomécanique de démarche est sélectionné parmi : vitesse de propulsion, taux de fatigue, angle de Fick, direction de propulsion et direction de décélération. De tels paramètres n'ont jamais été proposés dans le cadre de caractérisation de la démarche avec des capteurs de mouvement inertiels tels que dans le procédé selon l'invention. De tels paramètres dont l'accès est possible grâce au procédé selon l'invention permettraient d'ouvrir de nouvelles voies de caractérisation de la démarche.

- les moments clés comportent : une pose du talon, une pose des orteils, une levée du talon et/ou un décollage des orteils. Ces moments clés sont les plus informatifs.

- il comporte en outre une étape de prétraitement des valeurs d'accélération, de vitesse angulaire et d'orientation de la semelle, ladite étape de prétraitement comportant au moins un traitement sélectionné parmi : un filtrage fréquentiel, une suppression de la gravité sur les valeurs d'accélération, une suppression de la gravité, une suppression du bruit et/ou de la dérive sur les valeurs d'accélération, de vitesse angulaire et d'orientation de la semelle.

- l'étape de prétraitement est réalisée dans un boitier électronique intégré à la semelle. Cette étape de prétraitement peut être réalisée dans un dispositif électronique externe tel qu'un téléphone portable ou encore un serveur informatique. Néanmoins l'étape de prétraitement est de préférence réalisée dans un boitier électronique intégré à la semelle. C'est à dire que le boitier électronique est de préférence dans la semelle et non fixé à une cheville ou fixé sur le dessus d'une chaussure. Cela permet d'augmenter la précision du procédé.

- Il comporte en outre une étape d'identification d'une unité de mouvement. Une telle étape peut être mise en oeuvre par les différentes méthodes connues de la personne du métier.

- l'étape d'identification d'une activité associée à une unité de mouvement comporte le calcul d'un ou plusieurs paramètres descriptifs globaux à partir des valeurs d'accélération, de vitesse angulaire et/ou de champs magnétique, lesdits paramètres descriptifs globaux comportant une valeur de kurtosis ou de coefficient d'asymétrie. De préférence les paramètres descriptifs globaux sont calculés à partir des valeurs d'accélération et de vitesse angulaire. De tels paramètres descriptifs globaux permettent de discriminer plus efficacement les activités.

- l'étape d'identification d'une activité associée à une unité de mouvement comporte le calcul d'un ou plusieurs paramètres descriptifs globaux à partir des valeurs d'accélération, de vitesse angulaire et/ou de champs magnétique, lesdits paramètres descriptifs globaux comportant une ou plusieurs valeurs de coefficients de similarité avec des motifs prédéterminés. L'utilisation de motifs prédéterminés permet d'augmenter la précision de la méthode.

- l'étape d'identification d'une activité associée à une unité de mouvement comporte une étape de reconnaissance probabiliste et/ou une étape de reconnaissance déterministe. La présence d'une ou de plusieurs de ces étapes permet d'identifier avec plus de certitude l'activité associée à l'unité de mouvement.

- l'étape d'identification d'au moins deux moments clés est réalisée dans un boitier électronique intégré à la semelle. Cette étape d'identification peut être réalisée dans un dispositif électronique externe tel qu'un téléphone portable ou encore un serveur informatique. Néanmoins, elle est de préférence réalisée dans un boitier électronique intégré à la semelle. De même, l'étape de détermination d'une valeur de paramètre biomécanique avancé de marche est réalisée dans un boitier électronique intégré à la semelle. Néanmoins dans certains cas et par exemple pour certains paramètres biomécaniques avancés elle peut avantageusement être réalisée dans un dispositif électronique externe.

- il comporte en outre une étape de calcul d'un motif combinatoire de valeurs de paramètres biomécaniques avancés, ledit motif combinatoire de valeurs de paramètres biomécaniques avancés correspondant à une combinaison de valeurs de paramètres biomécaniques avancés ou à une combinaison de comportement en fonction du temps de paramètres biomécaniques avancés. Un tel motif combinatoire de paramètres biomécaniques peut être avantageusement associé à un état physiologique de l'utilisateur. Cela est particulièrement avantageux car cela permet de générer de nouveaux motifs pouvant être corrélés à des états physiologiques ou des états pathologiques prédéterminés et ainsi accéder à partir d'une caractérisation de la démarche, à des données de risque pour l'utilisateur.

L'invention porte en outre sur un système de détermination d'une valeur de paramètre biomécanique avancé de démarche à partir de données générées par un ou plusieurs capteurs de mouvement d'une semelle, caractérisé en ce qu'il comporte :

- Deux ensembles d'un ou de plusieurs capteurs de mouvement d'une semelle, un premier ensemble étant apte à être associé à une première semelle ou chaussure et un second ensemble étant apte à être associé à une seconde semelle ou chaussure, chaque ensemble étant configurés pour générer des données comportant des valeurs d'accélération, de vitesse angulaire et/ou de champs magnétique ; et

- Un ou plusieurs modules d'analyse configurés pour déterminer d'une valeur de paramètre biomécanique avancé de démarche, lesdits un ou plusieurs modules d'analyse étant configurés pour :

  -- Acquérir les données générées par les un ou plusieurs capteurs de mouvement ;
  -- Calculer une valeur d'orientation de la semelle, ladite valeur d'orientation de la semelle étant calculée par rapport à un référentiel terrestre à partir de données comportant les valeurs d'accélération, de vitesse angulaire et/ou de champs magnétique ;
  -- Identifier une activité associée à une unité de mouvement, ladite identification d'une activité comportant la comparaison de valeurs de référence d'activité à des valeurs sélectionnées parmi : les valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle ;
  -- Identifier au moins deux moments clés de l'unité de mouvement pour laquelle une activité a été identifiée, ladite étape d'identification de moment clés comportant la comparaison de valeurs de paramètres de mouvement à au moins un seuil prédéterminé, ledit seuil prédéterminé étant associé à l'activité identifiée et lesdites valeurs de paramètres de mouvement comportant les valeurs d'accélération, de vitesse angulaire et/ou d'orientation de la semelle ; et

- Déterminer une valeur de paramètre biomécanique avancé de marche, ladite détermination comportant le calcul d'une valeur de paramètre biomécanique de démarche pour au moins un des moments clés identifiés et à partir des valeurs d'accélération, de vitesse angulaire et/ou d'orientation de la semelle.

En outre, la paire d'ensemble d'un ou de plusieurs capteurs de mouvement d'une semelle peut correspondre à une paire de boitiers électroniques aptes à être intégrés dans une paire de semelles.

Il peut avantageusement comporter plusieurs modules de traitement configurés pour chacun exécuter une partie de la détermination d'une valeur de paramètre biomécanique avancé de démarche.

D'autres avantages et caractéristiques de l'invention apparaitront à la lecture de la description suivante donnée à titre d'exemple illustratif et non limitatif, en référence aux Figures annexées :

La figure 1 représente un schéma représentatif d'un procédé selon un mode de réalisation de l'invention.

La figure 2 représente une vue en coupe longitudinale du dessus de deux semelles chacune contenant une cavité qui va laisser place à un boitier, chacune des antennes des boitiers étant située sur l'arrête extérieure vis à vis de chaque pied, selon un mode de réalisation de l'invention

La figure 3 représente un boitier électronique ouvert vu du dessus comprenant notamment une carte électronique, une batterie rechargeable, un connecteur et une antenne.

La figure 4 représente un boitier électronique, en vue éclatée en coupe de profil, comprenant notamment une batterie rechargeable, une carte électronique ainsi qu'une enveloppe extérieure en deux parties.

La figure 5 représente un schéma représentatif d'un procédé selon un mode de réalisation de l'invention où une partie des calculs sont réalisé dans des boitiers électroniques associés à des chaussures, par exemple intégrés dans des semelles.

La figure 6 représente un schéma représentatif d'un procédé selon un mode de réalisation de l'invention où les calculs sont réalisés dans un terminal externe.

Des aspects de la présente invention sont décrits en référence à des organigrammes et / ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. A cet égard, chaque bloc dans les organigrammes ou blocs-diagrammes peut représenter un système, un dispositif, un module ou un code, qui comprend une ou plusieurs instructions exécutables pour mettre en oeuvre la ou les fonctions logiques spécifiées.

Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et / ou de l'organigramme, et des combinaisons de blocs dans les schémas de principe et / ou l'organigramme, peuvent être mis en oeuvre par des systèmes matériels spéciaux qui exécutent les fonctions ou actes spécifiés ou effectuer des combinaisons de matériel spécial et d'instructions informatiques.

**[Description de l'invention]**

Dans la suite de la description, la « démarche » au sens de l'invention correspond à la posture, les mouvements, la locomotion, et l'équilibre de l'utilisateur. L'équilibre correspond notamment à l'équilibre postural lié à la stabilité du corps et plus particulièrement à la stabilité du centre de gravité d'un utilisateur. Néanmoins il peut intégrer aussi bien l'équilibre statique que l'équilibre dynamique.

La « caractérisation de la démarche » correspond, au sens de l'invention, à l'attribution d'une ou de plusieurs valeurs par exemple un score, un classement ou une note à une trajectoire ou au déplacement d'un pied d'un utilisateur. Cette caractérisation de la démarche permet d'obtenir une ou plusieurs valeurs numériques ou alphanumériques de paramètres bio-mécanique représentatifs de la démarche.

Par « paramètre bio-mécanique », on entend au sens de l'invention une caractéristique de la démarche de l'utilisateur. Par « paramètre bio-mécanique avancé », on entend au sens de l'invention une caractéristique de la démarche de l'utilisateur déterminé à un moment clé d'un cycle et donc plus complexe à déterminer.

On entend par « semelle » un objet permettant de séparer le pied de l'utilisateur du sol. Une chaussure peut comporter une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible.

On entend par « amovible » la capacité à être détachée, enlevée ou démontée aisément sans avoir à détruire des moyens de fixation soit parce qu'il n'y a pas de moyen de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

Par « modèle » ou « règle » ou « algorithme », il faut comprendre au sens de l'invention une suite finie d'opérations ou d'instructions permettant de calculer une valeur par l'intermédiaire d'un classement ou d'un partitionnement des données au sein de groupes préalablement définis Y et d'attribuer un score ou de hiérarchiser une ou plusieurs données au sein d'un classement. La mise en oeuvre de cette suite finie d'opérations permet par exemple d'attribuer une étiquette Y à une observation décrite par un ensemble de caractéristiques ou paramètres X grâce par exemple à la mise en oeuvre d'une fonction f, susceptible de reproduire Y en ayant observé X.

$$Y = f(X) + e$$

où e symbolise le bruit ou erreur de mesure

Par « méthode d'apprentissage supervisé », on entend au sens de l'invention un procédé permettant de définir une fonction f à partir d'une base de n observations étiquetées $(X_{1...n}, Y_{1...n})$ où $Y = f(X) + e$.

Par « méthode d'apprentissage non supervisée », on entend une méthode visant à hiérarchiser les données ou à diviser un ensemble de données en différents groupes homogènes, les groupes homogènes partageant des caractéristiques communes et cela sans que les observations soient étiquetées.

On entend par « sensiblement constante » une valeur variant de moins de 30 % par rapport à la valeur comparée, de préférence de moins de 20 %, de façon encore plus préférée de moins de 10 %.

On entend par « traiter », « calculer », « déterminer », « afficher », « transformer », « extraire », « comparer » ou plus largement « opération exécutable », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou tout autre séquence d'instructions conçues pour l'exécution sur un système informatique.

On entend par « processeur », au sens de l'invention, au moins un circuit matériel configuré pour exécuter des opérations selon des instructions contenues dans un code. Le circuit matériel peut être un circuit intégré. Des exemples d'un processeur comprennent, sans s'y limiter, une unité de traitement central, un processeur graphique, un circuit intégré spécifique à l'application (ASIC) et un circuit logique programmable.

On entend par « couplé », au sens de l'invention, connecté, directement ou indirectement avec un ou plusieurs éléments intermédiaires. Deux éléments peuvent être couplés mécaniquement, électriquement ou liés par un canal de communication.

Dans cette description et même avant, les mêmes références sont utilisées pour désigner les mêmes éléments.

Pour rappel, les deux pieds contiennent le quart de tous les os du corps humain. Dans chaque pied, il est possible d'identifier 26 os, 33 muscles, 16 articulations et 107 ligaments. Les pieds portent le poids du corps en position debout et permettent la locomotion, assurant un rôle primordial dans l'équilibre, l'amortissement et la propulsion. Les pieds effectuent aussi plusieurs types de mouvements. En outre, les pieds comptent près de 7 200 terminaisons nerveuses, de sorte que toutes les maladies et autres troubles notamment neurologiques sont perceptibles directement ou indirectement au niveau de nos pieds et, d'autre part, peuvent être détectés à partir de notre manière de marcher ou de nous mouvoir.

Les inventeurs ont testé plusieurs procédés afin de quantifier la posture et la démarche d'un utilisateur. Ils ont sélectionné les systèmes intégrant des capteurs inertiels, systèmes les plus à même de fournir une réponse rapide et à un coût supportable pour une industrialisation. En effet, les procédés existants se basent généralement sur des capteurs de pression ou sur une pluralité de capteurs répartis dans la chaussure et/ou semelle ou même sur la cheville. Une telle répartition des capteurs entraine une réduction de la robustesse du système.

Néanmoins la plupart des procédés testés basés sur des données de capteurs inertiels (accélération et vitesse angulaire) présentent des biais trop importants lors du calcul de certaines valeurs de paramètres biomécaniques avancés, ce qui limite leur utilisation comme critères de caractérisation de la démarche.

En effet, les inventeurs ont lors d'essais des procédés de traitement de données de plateforme inertielle habituels, mis en exergue comme d'autres, une variabilité de la marche interpersonnelle et intrapersonnelle pouvant, lorsque les traitements de données ne sont pas adaptés, entrainer des biais préjudiciables. De plus, avec les procédés de traitement faisant appel à des analyses de motif sur fenêtre glissante à partir des données brutes des deux pieds, la consommation en énergie et en mémoire réduisaient l'autonomie du dispositif à quelques dizaines de minutes. Cela, même lorsque les calculs étaient mis en oeuvre sur des téléphones, des montres connectés ou encore des serveurs distants.

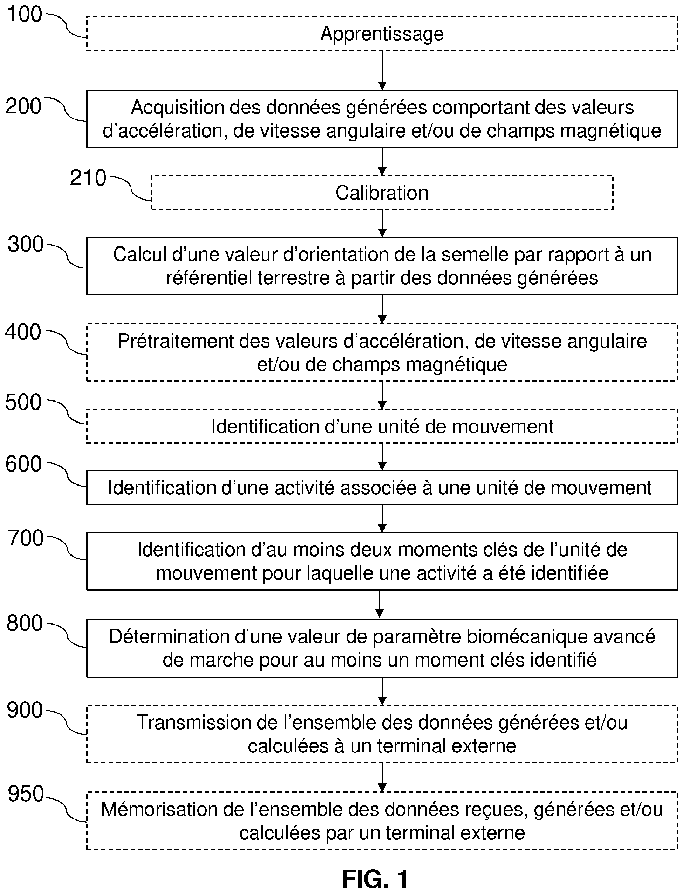

Face à ces insuffisances, l'inventeur a développé un procédé 1 de détermination d'une valeur de paramètre biomécanique avancé de démarche à partir de données générées par un ou plusieurs capteurs de mouvement d'une semelle tel que schématisé sur la Figure 1.

**Selon un premier aspect,** l'invention porte sur un **procédé de détermination d'une valeur de paramètre biomécanique avancé de démarche.** En particulier, un tel procédé est mis en oeuvre par un ou plusieurs modules d'analyse. De préférence, par plusieurs modules d'analyses, chacun étant configuré pour exécuter une partie du procédé.

En particulier, un tel procédé est mis en oeuvre à partir de données générées par un ou plusieurs capteurs de mouvement d'une semelle, décrits par la suite. Certains des capteurs de mouvement peuvent être répartis dans la semelle ou bien aussi être positionnés sur la chaussure de façon à suivre les mouvements de la semelle. Les capteurs de mouvement peuvent aussi être tous intégrés dans un premier boitier associé à une première chaussure et un deuxième boitier associé à une deuxième chaussure de la même paire.

Comme cela est illustré à la **figure 1**, un tel procédé selon l'invention comporte en particulier les étapes suivantes : acquisition 200 de données générées par un ou plusieurs capteurs de mouvement d'une semelle, calcul 300 d'une valeur d'orientation de la semelle, identification 600 d'une activité associée à une unité de mouvement, identification 700 d'au moins deux moments clés de l'unité de mouvement pour laquelle une activité a été identifiée, et détermination 800 d'une valeur de paramètre biomécanique avancé de marche.

En outre, un procédé selon l'invention peut comporter des étapes avantageuses telles que : un apprentissage 100 de la démarche de l'utilisateur, un prétraitement 400 des données générées par les capteurs de mouvement et/ou des valeurs calculées dans le cadre du procédé, une identification 500 d'une unité de mouvement, une transmission 900 de données ou encore une mémorisation 950 de données.

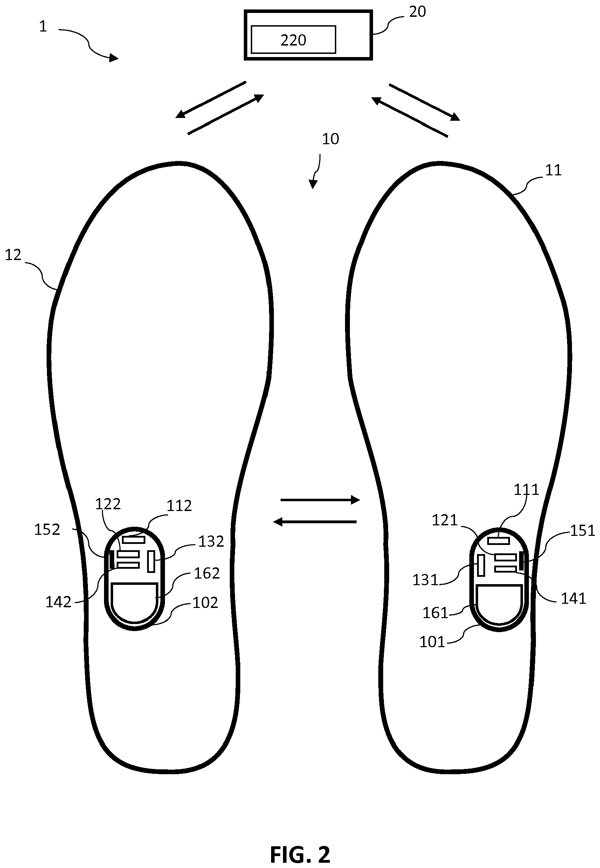

Comme illustré à la figure 2, un procédé selon l'invention peut comporter une **étape 100 d'apprentissage** de la démarche de l'utilisateur. Une telle étape peut notamment comporter la définition d'une pluralité des valeurs de référence d'activité d'un utilisateur. Par exemple, lors de la répétition de mouvements, postures et démarches effectués par un utilisateur sur une durée déterminée, des valeurs de référence d'activité peuvent être enregistrées et classées selon une pluralité de valeurs, incluant des motifs par exemple de type statique ou dynamique.

Ainsi, un certain motif dynamique peut représenter un mouvement d'un utilisateur tel qu'à titre d'exemple non limitatif, un « pas » et un motif statique peut, de manière avantageuse mais non limitative, représenter une posture de type « à genou » d'un utilisateur. En particulier, les motifs prédéterminés peuvent avoir été normalisés en fonction d'une période de temps correspondant à un cycle, le cycle pouvant par exemple être un cycle de marche. Il existe différents types d'activités telles que le pas, la montée d'une marche, la descente d'une marche, une foulée, un saut, un plat, un statisme, un piétinement, un agenouillement... De ce fait, un cycle peut également correspondre à une pluralité d'activités de types différents en fonction de la complexité du mouvement réalisé par l'utilisateur.

Cette étape 100 d'apprentissage peut être mise en oeuvre lors de la première utilisation des capteurs de mouvement par ledit utilisateur et elle peut également être répétée ponctuellement de façon à augmenter la précision de l'étape 600 d'identification d'une activité associée à une unité de mouvement.

Ainsi, un procédé conforme à l'invention peut comporter la génération de valeurs de référence chacune étant associée à une activité et plus particulièrement à un ensemble de valeurs de référence par activité. Le procédé peut alors comporter la génération de plusieurs ensembles de valeurs de référence de façon à couvrir plusieurs activités de l'utilisateur. De façon plus préférée, le procédé comporte la génération d'au moins quatre ensembles de valeurs de référence.

Les valeurs de référence d'activité d'un utilisateur pourront par exemple être stockées dans un boitier électronique, les valeurs de référence du pied droit étant stockées dans le boitier électronique associé au pied droit et les valeurs de référence du pied gauche étant stockées dans le boitier électronique associé au pied gauche.

Les valeurs de référence d'activité d'un utilisateur peuvent également être transmises à un terminal externe qui pourra les mémoriser ou les transmettre à d'autres terminaux.

Un procédé selon l'invention comporte une **étape 200 d'acquisition de données générées par un ou plusieurs capteurs de mouvement d'une semelle.** L'acquisition comme cela peut être compris à la lecture de la présente description est de préférence réalisée pour chacune des deux semelles. Cette étape peut correspondre en particulier dans le cadre de l'invention à l'acquisition de données générées par un ou plusieurs capteurs de mouvement d'un boitier ou de plusieurs boitiers par exemple chacun intégré à une semelle.

En particulier, les données acquises comportent des valeurs d'accélération, de vitesse angulaire et/ou de champs magnétique. De façon préférée, les données acquises comportent des valeurs d'accélération, de vitesse angulaire et de champs magnétique. Cette étape d'acquisition peut également comporter l'acquisition d'autres données telles que des données de géolocalisation, des données de température, des données de pression.

Le procédé selon l'invention se base au moins en partie sur des données générées par des centrales inertielles. Ainsi, il peut comporter une étape de génération de données brutes des capteurs de mouvement d'une semelle comportant une ou plusieurs plateformes inertielles. Ces données brutes sont généralement générées pour une période de temps donné et en fonction de la démarche d'un utilisateur.

En particulier, les plateformes inertielles peuvent être contenues dans un premier et un second boitier. Les données brutes peuvent ainsi être issues d'un gyroscope, d'un accéléromètre et d'un magnétomètre, par exemple contenus dans chaque boitier. En outre, selon un mode de réalisation avantageux, les données brutes sont collectées par les deux boitiers sur une durée prédéterminée pouvant par exemple s'étaler de la milliseconde à plusieurs heures.

De façon préférée, les capteurs de mouvement, de préférence les boitiers électroniques, sont configurés pour communiquer entre eux et pour initier la génération de données sur le mouvement seulement après réception par un premier boitier électronique d'un message provenant de l'autre boitier électronique. Ainsi, les générations des données par les plateformes inertielles, par exemple des deux boitiers, sont de préférence synchronisées, ce qui permet une analyse plus fine et plus juste de la démarche de l'utilisateur. En absence d'une telle synchronisation, les paramètres biomécaniques avancés calculés à partir de données du premier boitier ne pourront pas être fusionnés avec une précision élevée avec des paramètres biomécaniques avancés calculés à partir de données du second boitier pour calculer de nouveaux paramètres biomécaniques avancés de mouvement.

En particulier, un procédé selon l'invention peut comprendre **une étape de calibration 210,** entre un premier et un second ensemble de capteurs de mouvement. Comme cela sera décrit par la suite, chacun des ensembles de capteurs de mouvement peut être associé à une chaussure et peut prendre la forme d'un premier et d'un deuxième boitier électronique.

L'étape de calibration peut comporter l'émission d'un signal par le premier ensemble de capteurs de mouvement et la réception du signal émis par le second ensemble de capteurs de mouvement afin de calibrer un moyen de mesure de temps, de préférence une horloge. Un tel calibrage permet au premier et au second boitier de détecter et collecter des données de posture ou de démarche d'un utilisateur sur une même fenêtre temporelle. En effet, les données collectées par le premier et le second boitier peuvent être analysées deux à deux en particulier pour certains paramètres biomécaniques avancés de démarche. Ainsi, les données collectées seront analysées en parallèle ce qui permet d'éviter tout décalage entre les données et toutes erreurs d'analyse.

Un procédé selon l'invention comporte également **une étape 300 de calcul d'une valeur d'orientation de la semelle.**

L'orientation de la semelle correspond en particulier aux positions angulaires de la semelle selon trois axes orthogonaux x, y et z. Ainsi, de façon préférée, l'étape 300 de calcul d'une valeur d'orientation de la semelle permet de calculer une valeur de position angulaire pour chacun des trois axes x, y et z.

La valeur d'orientation de la semelle peut être calculée par rapport à un référentiel terrestre et à partir de données comportant les valeurs d'accélération, de vitesse angulaire et/ou de champs magnétique. En particulier, elle peut être calculée à partir des valeurs d'accélération, de vitesse angulaire et de champs magnétique.

De nombreux algorithmes, tels que des algorithmes de fusion, peuvent être utilisés pour calculer la valeur d'orientation de la semelle.

Par exemple, un procédé conforme à l'invention peut mettre en oeuvre un algorithme de fusion des données de l'accéléromètre, du gyroscope et du magnétomètre sur la base de filtres couplés à des modèles probabilistes. Alternativement, un procédé conforme à l'invention peut mettre en oeuvre un algorithme de fusion des données de l'accéléromètre et du gyroscope sur la base de filtres couplés à des modèles probabilistes.

Un tel procédé peut aussi mettre en oeuvre un algorithme de type filtre de Kalman et ses variantes. Ainsi peuvent par exemple être utilisés : un filtre de Kalman linéaire, un filtre de Kalman étendu, un filtre de Kalman étendu additif, un filtre de Kalman étendu multiplicatif, un filtre de Kalman à cubage ou un filtre particulaire.

Alternativement, un procédé selon l'invention peut aussi mettre en oeuvre un algorithme de fusion de Premerlani & Bizard, un algorithme de fusion de Mahony ou un algorithme de fusion de Madgwick.

En outre, un tel procédé peut mettre en oeuvre des matrices de rotation formées sur la base de cosinus directeurs ou de quaternions.

**Un procédé selon l'invention peut comporter une étape 400 de prétraitement des données** générées par un ou plusieurs capteurs de mouvement d'une semelle. En outre, cette étape de prétraitement peut concerner des valeurs calculées dans le cadre de la mise en oeuvre d'un procédé selon l'invention, comme par exemple les valeurs d'orientation de la semelle.

En particulier cette étape de prétraitement peut correspondre au prétraitement des valeurs d'accélération, de vitesse angulaire et d'orientation de la semelle. Par exemple elle peut comporter en particulier au moins un traitement sélectionné parmi : un filtrage fréquentiel, une suppression de la gravité sur les valeurs d'accélération, une suppression de la gravité, une suppression du bruit sur les valeurs d'accélération et une suppression de la dérive sur les valeurs de vitesse angulaire et/ou d'orientation de la semelle.

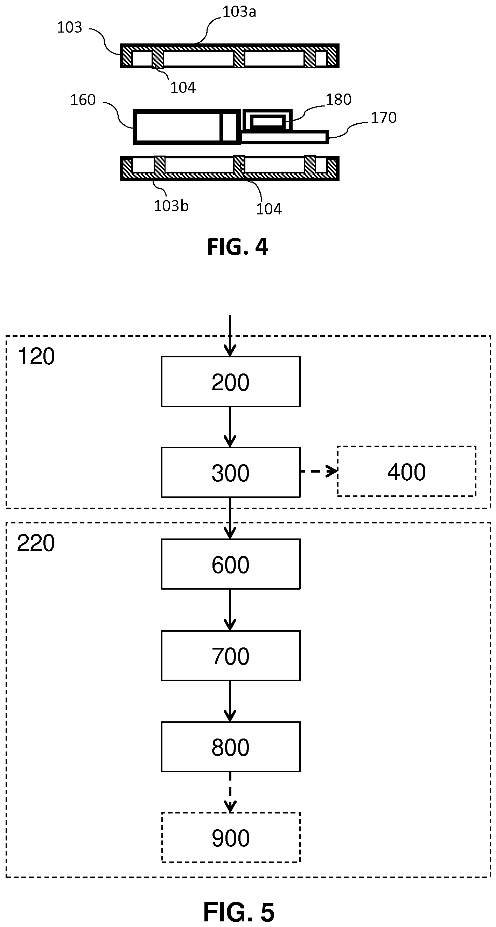

Comme cela est illustré à la figure 5, cette étape 400 de prétraitement peut être mise en oeuvre par des modules de traitement de données contenus dans les capteurs de mouvement et plus particulièrement dans le premier et le second boitier.

Alternativement, cette étape 400 de prétraitement peut être mise en oeuvre par un module de traitement de données contenu dans un terminal externe 20.

Un procédé selon l'invention peut également comporter **une étape 500 d'identification d'une unité de mouvement. L'unité de mouvement au sens de**

**l'invention correspond à un cycle de marche, un cycle de course ou encore un cycle de montée d'escalier.**

Ces cycles peuvent être définis comme la période de temps allant du contact initial d'un pied à l'occurrence suivante du même événement avec le même pied.

Dans le cadre de l'invention de nombreuses méthodes de partitionnement peuvent être utilisées. Ainsi cette étape peut par exemple comporter la mise en oeuvre d'étapes classiques d'étude du mouvement du pas comme par exemple la méthode Pan-Tompkins ou la détection de seuils, de maxima et/ou de minima.

Un procédé selon l'invention comporte également une **étape 600 d'identification d'une activité associée à une unité de mouvement.**

Cette identification peut par exemple être réalisée par un module de traitement d'un terminal externe. Une telle étape permet de filtrer les données de façon à ne calculer dans les prochaines étapes des paramètres biomécanique avancés que sur des unités de mouvement qualifiées/confirmées. Ainsi, la justesse de la mesure en est améliorée.

Cette identification d'une activité comporte de préférence la comparaison de valeurs de référence d'activité à des valeurs sélectionnées parmi : des valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle.

Les valeurs de référence d'activités peuvent être les valeurs de référence d'activité générées lors de l'étape 100 d'apprentissage, par exemple dans le cas d'une reconnaissance déterministe et/ou des valeurs de référence d'activité prédéterminées par exemple dans le cas d'une reconnaissance probabiliste.

Les paramètres descriptifs globaux calculés à partir des valeurs d'accélération, de vitesse angulaire et/ou d'orientation de la semelle peuvent par exemple être une variance, une moyenne une médiane, un kurtosis ou un coefficient d'asymétrie.

En particulier, l'étape 600 d'identification d'une activité associée à une unité de mouvement comporte le calcul d'un ou plusieurs paramètres descriptifs globaux à partir des valeurs d'accélération, de vitesse angulaire et/ou de champs magnétique, par exemple à partir des valeurs d'accélération et de vitesse angulaire ou à partir des valeurs d'accélération, de vitesse angulaire et de champs magnétique. Les paramètres descriptifs globaux comportant de préférence une valeur de kurtosis ou de coefficient d'asymétrie. Néanmoins, les paramètres descriptifs globaux peuvent comporter d'autres valeurs ou coefficients. Par exemple, les paramètres descriptifs globaux peuvent correspondre de façon préférées à des valeurs de coefficients de similarité avec des motifs prédéterminés.

En outre, l'étape 600 d'identification d'une activité associée à une unité de mouvement peut comporter une étape de reconnaissance probabiliste et/ou une étape de reconnaissance déterministe.

De façon préférée, l'étape 600 d'identification d'une activité associée à une unité de mouvement comporte une étape de reconnaissance probabiliste et une étape de reconnaissance déterministe. En effet, la reconnaissance déterministe permet de coller au mieux au déplacement de la personne, et la reconnaissance probabiliste permet d'enrichir aisément les possibilités de reconnaissance de façon à permettre une adaptation au besoin de l'utilisateur.

L'étape de reconnaissance probabiliste se base sur des modèles préétablis et l'identification de l'activité intègre alors un calcul de probabilité qu'une unité de mouvement corresponde à une activité plutôt qu'à une autre.

La reconnaissance probabiliste comporte par exemple une sous-étape de calcul de paramètres descriptifs globaux permettant de décrire le mouvement de la semelle, une sous-étape de comparaison des paramètres descriptifs globaux calculés à un modèle d'apprentissage préétabli et une identification de l'activité en fonction des résultats de la comparaison.

Le modèle d'apprentissage préétabli peut par exemple être établi via des approches supervisées ou non supervisées. Parmi les méthodes d'apprentissage supervisées, les réseaux de neurones, les arbres de classification ou les arbres de régression sont parmi les techniques d'apprentissage automatique les plus robustes et les plus efficaces dans le cadre du procédé selon l'invention.

Il est par exemple possible d'utiliser une étape d'apprentissage supervisé pour apprendre à des arbres à classer les unités de mouvement en fonction d'une activité associée. Pour cela, le procédé comporte de préférence une étape préalable de réception de valeurs étiquetées (avec l'activité associée) telles que des valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle.

Dans un mode de réalisation préférée, un apprentissage supervisé est utilisé pour entrainer un algorithme de type CART (« Classification And Regression Trees » en terminologie anglosaxonne). L'algorithme de type CART peut alors être configuré pour prédire l'activité à partir des valeurs sélectionnées et à calculer un intervalle de confiance ou une erreur de prédiction. Alternativement, un réseau de neurone de type LSTM (« Long short-term memory » en terminologie anglosaxonne) peut aussi être utilisé pour établir le modèle d'apprentissage préétabli.

L'étape de reconnaissance déterministe se base sur des mouvements de référence réalisés par l'utilisateur lors de l'étape d'apprentissage. L'étape de reconnaissance déterministe peut alors intégrer la génération d'un score de ressemblance.

En outre, l'étape 600 d'identification d'une activité associée à une unité de mouvement peut comporter une étape de calcul d'une valeur de similarité entre chacune des valeurs de référence d'activité et les valeurs sélectionnées pour l'identification.

Ainsi, l'étape 600 d'identification d'une activité associée à une unité de mouvement pourra comporter la réalisation d'un classement entre les valeurs de référence de façon à déterminer la ou les activités pouvant correspondre à l'unité de mouvement étudiée d'une part et le degré de similitude d'autre part. En outre, l'étape 600 d'identification pourrait comporter une étape de validation de l'unité de mouvement lorsque cette dernière permet la génération d'une valeur de similarité dépassant un seuil prédéterminé.

Une valeur de similarité peut être calculée pour les données générées du premier et du second boitier. Ces calculs peuvent par exemple être réalisés par un terminal externe 20. Avantageusement, les données étant synchronisées, une valeur de similarité est calculée, pour une même période de temps, une première fois pour les données du premier boitier et une seconde fois pour les données du second boitier. Ainsi il est possible de quantifier la posture ou la démarche d'un utilisateur de façon plus fiable et dont la précision est améliorée.

Ainsi, le procédé peut comporter une étape de sélection d'une activité représentative d'une unité de mouvement à partir des valeurs de similarités. Particulièrement, cette étape comprend la sélection d'une activité si, pour un même moment temporel et étant donné que les mesures de données sont préférentiellement synchronisées, une première activité représentative d'une unité de mouvement d'un premier ensemble de capteurs est identique à une seconde activité représentative d'une unité de mouvement d'un second ensemble de capteurs. Ceci permet de prendre les données des deux pieds avant de sélectionner une activité et permet d'augmenter encore la précision du procédé.

En outre, si aucune valeur de similarités ne dépasse un seuil prédéterminé, le procédé peut comporter une non prise en considération de cette unité de mouvement car ne correspondant pas à une activité bien caractérisée et donc susceptible d'apporter une imprécision aux mesures de paramètres biomécaniques avancés réalisées.

Le procédé selon l'invention comporte une **étape 700 d'identification d'au moins deux moments clés de l'unité de mouvement pour laquelle une activité a été identifiée.** En particulier, l'étape d'identification de moment clés comportant la comparaison des valeurs d'orientation de la semelle à au moins un seuil prédéterminé. De façon préférée, l'au moins un seuil prédéterminé est associé à l'activité identifiée. Ainsi, en fonction de l'activité identifiée, le ou les seuils prédéterminés pourront être modifiés de façon à permettre une analyse plus fine et un calcul plus juste de valeurs de paramètre biomécaniques avancés.

Alors que la plupart des procédés de l'art antérieur cherchent à déterminer des seuils prédéterminés ou des algorithmes permettant de s'adapter à tous les cycles, la présente invention se base sur une combinaison d'identification d'activité à une sélection de moments clés basé sur un ou plusieurs seuils prédéterminés spécialement adaptés à cette activité.

Un seuil prédéterminé associé une activité identifiée peut par exemple correspondre à l'identification du dépassement d'une valeur prédéterminée ou alors à l'identification d'un passage d'une valeur positive à une valeur négative ou encore correspondre à l'identification d'un motif particulier d'une série de valeur indicatrice d'un moment clé. Ainsi, un seuil prédéterminé peut correspondre à l'identification d'un deuxième minima local, d'un maximum local ou encore à un enchainement d'un minima local et d'un maxima.

Les valeurs de paramètres de mouvement comportent par exemple les valeurs d'accélération, de vitesse angulaire et/ou d'orientation de la semelle.

Dans le cadre de cette étape 700 d'identification d'au moins deux moments clés de l'unité de mouvement, de nombreux modèles de partitionnement, chacun avec différents niveaux de granularité, peuvent être utilisés. Il est par exemple possible de découper le mouvement en deux phases principales, à savoir la posture et le basculement. Néanmoins, il est possible de découper en un plus grand nombre de phases. Par exemple, trois, quatre, cinq, six phases ou plus peuvent être considérées pour s'adapter à des utilisations particulières.

De préférence, le procédé comporte une étape 700 d'identification d'au moins quatre moments clés de l'unité de mouvement pour laquelle une activité a été identifiée. Le moment de l"impact correspondant au moment précis du contact du pied (e.g. talon) avec le sol, la pose des orteils, la levée du talon et le décollage des orteils. De tels moments clés permettent d'identifier des phases telles que la phase d'appui (se déroule depuis la phase d'impact jusqu'au décollement du talon du sol), la phase de propulsion (débute quand le talon a quitté le sol et se termine quand le premier orteil a quitté le sol) et la phase de vol (débute quand le premier orteil a quitté le sol et se termine quand le talon touche le sol).

Le procédé comporte également une **étape 800 de détermination d'une valeur de paramètre biomécanique avancé de démarche.**

De façon préférée, la détermination comporte un calcul d'un paramètre biomécanique de démarche pour au moins un des moments clés identifiés à partir des valeurs d'accélération, de vitesse angulaire et/ou d'orientation de la semelle. Le calcul pourra se faire à partir des valeurs d'accélération, de vitesse angulaire et/ou d'orientation de la semelle qui pourront être transformées (e.g. filtrées, corrigées...).

Le paramètre biomécanique avancé de démarche peut avantageusement être sélectionné parmi : vitesse de propulsion, taux de fatigue, angle de Fick, direction de propulsion et direction de décélération.

En particulier, la détermination peut comporter le calcul, pour au moins un moment clé identifié, d'une valeur de vitesse de la semelle à partir des valeurs accélérations audit au moins un moment clé identifié. De façon préférée, la détermination peut comporter le calcul, pour au moins un moment clé identifié, d'une valeur de vitesse de la semelle à partir des valeurs accélérations audit au moins un moment clé identifié et ledit moment clé identifié correspond au décollage des orteils. Ainsi, le procédé selon l'invention est en mesure de calculer la vitesse de propulsion, de préférence au niveau de chacun des pieds de l'utilisateur. Une telle vitesse de propulsion, au moment du décollage des orteils, est un paramètre biomécanique clé dans la caractérisation de la démarche et n'est pas proposé par les procédés existants.

L'angle de Fick correspond en particulier aux angles d'ouverture par rapport à la direction de la marche et entre les deux pieds. L'angle de Fick dans le cadre de la présente invention correspond aux valeurs calculées pour au moins deux moment clés, de préférence au moins trois moments clés et de façon encore plus préférée quatre moments clés.

La direction de propulsion correspond en particulier à l'angle entre la direction de décollage et l'axe du pied au décollage des orteils. La direction de propulsion dans le cadre de la présente invention correspond aux valeurs calculées pour au moins deux moment clés, de préférence au moins trois moments clés et de façon encore plus préférée quatre moments clés.

La direction de décélération correspond en particulier à l'angle entre la direction de décélération et l'axe du pied à la pose du talon. La direction de décélération dans le cadre de la présente invention correspond aux valeurs calculées pour au moins deux moment clés, de préférence au moins trois moments clés et de façon encore plus préférée quatre moments clés.

En outre, il a été déterminé dans le cadre de la présente invention que la relation entre la vitesse de propulsion (i.e. au moment du décollage des orteils) et la vitesse de la semelle au cours d'une phase de vol est indicatrice d'une fatigue de l'utilisateur. Ainsi, un procédé selon l'invention comporte aussi le calcul d'un taux de fatigue, ledit taux de fatigue correspondant à un rapport entre la vitesse de propulsion et la vitesse de la semelle au cours de la phase de vol.

En outre, le procédé selon l'invention peut comporter également une étape de calcul de valeurs de nouveaux paramètres biomécaniques avancés de démarche à partir de valeur de paramètres biomécaniques avancés de démarche obtenue à partir d'un premier ensemble de capteurs de mouvements associés au pied droit et de valeur de paramètres biomécaniques avancés de démarche obtenue à partir d'un second ensemble de capteurs de mouvements associés au pied gauche.

Ainsi, le procédé selon l'invention est apte à calculer, à partir des signaux générés par des plateformes inertielles, des paramètres biomécaniques avancés précis, représentatifs de la démarche de l'utilisateur. Comme cela sera détaillé par la suite, le calcul de ces paramètres biomécaniques avancés peut être réalisé en partie dans un boitier électroniques embarqué dans une semelle mais également intégralement dans un terminal externe. Alternativement, le calcul de ces paramètres biomécaniques avancés peut être réalisé intégralement dans un ou plusieurs boitiers électroniques embarqués.

De façon plus préférée, le procédé selon l'invention peut être utilisé pour calculer les valeurs d'au moins un, par exemple d'au moins deux, des paramètres biomécaniques suivants : la stabilité du pied lors de la phase de vol, le déroulé du pas, la longueur du pas, la largeur du pas, l'angle du pas, la longueur de la foulée, et/ou la largeur de la foulée.

Ceci constitue une liste de différents paramètres biomécaniques et l'invention ne se limite pas au calcul de ces paramètres particuliers. En effet, à partir des données générées par les plateformes inertielles, l'invention permet de calculer une pluralité de paramètres biomécaniques différents dont la liste n'est limitée que par leur utilité pour l'utilisateur.

Par exemple, le procédé peut permettre de calculer une valeur d'orientation de la propulsion. Ce paramètre biomécanique correspond plus particulièrement à l'angle d'un pied, par exemple par rapport au sol, lors de la phase de propulsion. De façon similaire, le procédé peut permettre de calculer une valeur de nombreux autres paramètres biomécaniques.

En outre, dans le cadre de la présente invention, le procédé selon l'invention peut comporter une étape de calcul d'une valeur de paramètre biomécanique dit synchronisé. Au sens de l'invention, un paramètre biomécanique dit synchronisé est un paramètre biomécanique dont le calcul nécessite des données provenant de deux ensembles de capteurs de mouvement, chacun étant associé à une semelle d'une paire de chaussure. Par exemple, dans ce contexte, le procédé comporte une étape de calcul d'une valeur de paramètre biomécanique dit synchronisé à partir d'un ou plusieurs paramètres biomécaniques calculés par le premier boitier électronique et d'un ou plusieurs paramètres biomécaniques calculés par le second boitier électronique. Ce mode de réalisation est particulièrement avantageux car il permet d'accéder à des caractérisations fines de la démarche.

De plus, dans le cadre de la présente invention, le procédé selon l'invention peut comporter une étape de calcul d'un motif combinatoire de paramètres biomécaniques. Au sens de l'invention, un motif combinatoire de paramètres biomécaniques correspond à une combinaison de paramètres biomécaniques (i.e. valeurs) ou à une combinaison de comportement en fonction du temps de paramètres biomécaniques. Un tel motif combinatoire de paramètres biomécaniques peut être avantageusement associé à un état physiologique de l'utilisateur. Ce mode de réalisation est particulièrement avantageux car il permet de générer de nouveaux motifs pouvant être corrélés à des états physiologiques ou des états pathologiques prédéterminés et ainsi accéder à partir d'une caractérisation de la démarche, à des données de risque pour l'utilisateur. Alternativement, le calcul d'un motif combinatoire de paramètres biomécaniques puis sa comparaison peut être réalisé par le module de traitement porté par un terminal externe.

Un motif combinatoire de paramètres biomécaniques peut par exemple comporter en une combinaison d'une valeur de cadence, d'une valeur de longueur de foulée et d'une vitesse de marche. Un tel motif combinatoire de paramètres biomécaniques permet à partir des valeurs individuelles de chacun de ces trois paramètres de déterminer un désordre de la marche pouvant par exemple être causé par une aggravation d'un pas parkinsonien.

En outre, le procédé selon l'invention peut être configuré pour calculer une asymétrie entre les paramètres biomécaniques d'une jambe droite par rapport aux paramètres biomécaniques d'une jambe gauche.

En outre, le procédé selon l'invention peut être configuré pour calculer une variabilité des paramètres biomécaniques associés à une jambe ou aux deux jambes.

Avantageusement, le procédé selon l'invention comporte une étape de profilage consistant à établir un profil de l'utilisateur lors d'une première période d'utilisation. Cette première période d'utilisation peut par exemple présenter une durée d'une journée, d'une semaine ou encore d'un mois. Une première période d'utilisation présente de préférence une durée suffisante pour calculer un ensemble de paramètres biomécaniques avancés de démarche stables dans le temps avec de façon préférée une faible variabilité (e.g. inférieure à 20 %, de préférence inférieure à 10 %). Il faut généralement de quelques jours à quelques semaines pour établir un profil de l'utilisateur.

Avantageusement, si un des boitiers venait à se déconnecter ou perdre la synchronisation dans le temps par rapport à l'autre boitier, le procédé comprend **une étape de synchronisation des boitiers.** Ainsi, un signal de recherche est envoyé par le boitier connecté, le boitier déconnecté reçoit le signal de recherche et se synchronise sur le boitier connecté.

De préférence, le procédé peut également comporter une étape de transmission **900** de données à un terminal externe. Cette transmission se fait de préférence ponctuellement. En particulier cela peut correspondre à la transmission de l'ensemble des données générées et/ou calculées à un terminal externe.

En particulier, la transmission peut être réalisée par un module de communication des boitiers électroniques.

En outre, le procédé peut comporter la transmission des données reçues par le terminal externe à un ou d'autre terminaux externes qui pourraient être impliqués dans le calcul des valeurs de paramètres biomécaniques avancés de démarche ou dans leur affichage.

Les données transmises peuvent par exemple être des données brutes telles qu'elles ont été générées par les capteurs de mouvement, des données pré-traitées, ou encore des données calculées telle que par exemple des valeurs d'orientation de la semelle.

Le procédé peut également comporter une étape de mémorisation 950 de la valeur de paramètre biomécanique avancé de démarche. Il peut également comporter une mémorisation des valeurs générées par les capteurs de mouvement, des données pré-traitées, ou encore des données calculées telle que par exemple des valeurs d'orientation de la semelle. En particulier, cela peut correspondre à la mémorisation de l'ensemble des données reçues, générées et/ou calculées par un terminal externe.

Avantageusement, par opposition aux données brutes et/ou prétraitées qui sont sauvegardés sur une durée courte (e.g. inférieure à 5 minutes) par exemple sur une mémoire cache, une valeur de paramètre de démarche avancé est mémorisée à plus long terme, par exemple sur une mémoire.

Avantageusement, le calcul d'une ou de plusieurs valeurs de paramètres biomécanique avancé de démarche est fait en temps réel, c'est-à-dire moins de 1 heure après la génération des données par l'un ou plusieurs capteurs de mouvement d'une semelle, de préférence moins de 10 minutes, de façon plus préférée moins d'une minute, de façon encore plus préférée moins de 10 secondes.

**Selon un autre aspect,** l'invention porte sur un système 1 de détermination d'une valeur de paramètre biomécanique avancé de démarche à partir de données générées par un ou plusieurs capteurs de mouvement d'une semelle.

De façon préférée, un tel système comporte des modules configurés pour exécuter un procédé selon l'invention et ses différents modes de réalisation, préférés, avantageux ou non.

En particulier, le système selon l'invention comporte deux ensembles d'un ou de plusieurs capteurs de mouvement d'une semelle 101, 102, et un ou plusieurs modules d'analyse 120, 121, 122, 220 configurés pour déterminer d'une valeur de paramètre biomécanique avancé de démarche.

De préférence, le premier ensemble est apte à être associé à une première chaussure et un second ensemble étant apte à être associé à une seconde semelle, chaque ensemble étant configurés pour générer des données comportant des valeurs d'accélération, de vitesse angulaire et/ou de champs magnétique.

De préférence, le système comporte plusieurs modules d'analyse, deux modules d'analyse 120, 121, 122 pouvant être associés au niveau de la chaussure avec les capteurs de mouvement, par exemple au sein de deux boitiers électroniques et un autre module d'analyse 220 étant associé à un terminal externe 20.

Ces modules d'analyse sont configurés de façon à ensemble exécuter les actions suivantes :

- Acquérir les données générées par l'un ou plusieurs capteurs de mouvement.

- Calculer une valeur d'orientation de la semelle, ladite valeur d'orientation de la semelle étant calculée par rapport à un référentiel terrestre à partir de données comportant les valeurs d'accélération, de vitesse angulaire et/ou de champs magnétique

- Identifier une activité associée à une unité de mouvement, ladite identification d'une activité comportant la comparaison de valeurs de référence d'activité à des valeurs sélectionnées parmi : les valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle ;

- Identifier au moins deux moments clés de l'unité de mouvement pour laquelle une activité a été identifiée, ladite étape d'identification de moment clés comportant la comparaison de valeurs de paramètres de mouvement à au moins un seuil prédéterminé, ledit seuil prédéterminé étant associé à l'activité identifiée et lesdites valeurs de paramètres de mouvement comportant les valeurs d'accélération, de vitesse angulaire et/ou d'orientation de la semelle ; et

- Déterminer une valeur de paramètre biomécanique avancé de marche, ladite détermination comportant le calcul d'une valeur de paramètre biomécanique de démarche pour au moins un des moments clés identifiés et à partir des valeurs d'accélération, de vitesse angulaire et/ou d'orientation de la semelle.

Ces actions peuvent être réalisée en redondances entre les différents modules d'analyse.

En particulier, le système 1 selon l'invention peut comporter une paire 10 de semelles comportant les boitiers électroniques 101, 102 selon l'invention et éventuellement un terminal externe 20.

Les semelles 11, 12 utilisables dans le cadre du système 1 selon l'invention peuvent par exemple correspondre à des semelles extérieures ou à des semelles intérieures, de chaussures. Ces semelles peuvent être amovibles ou être intégrées de manière permanente au semelage des chaussures.

Classiquement, les semelles 11, 12 composants ladite paire 10 de semelles, comportent chacune un boitier électronique 101,102. Comme présenté sur la figure 2, le boitier électronique 101,102 est de préférence positionné au niveau d'une portion médiane de la semelle.

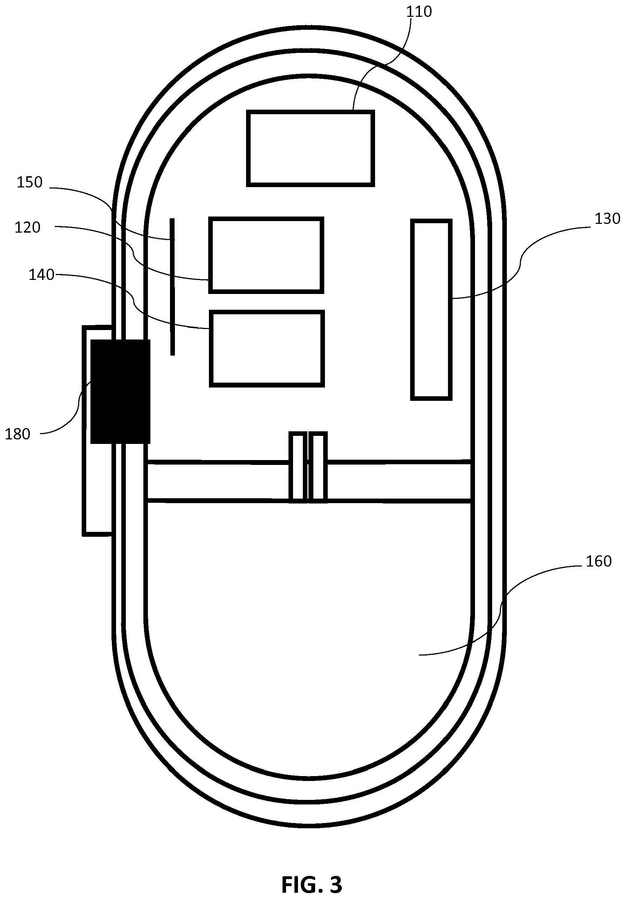

Un boitier électronique selon l'invention est détaillé dans la figure 3. Ne pesant que quelques grammes et étant de dimension réduite, ce boitier électronique se loge de façon peu encombrante dans n'importe quelle semelle intérieure et/ou extérieure. Ce faible volume limite l'impact sur le confort de l'utilisateur et présente l'avantage d'optimiser les coûts de production en rendant moins onéreux et plus simple l'intégration de cette technologie dans la semelle lors du processus industriel.

En outre, le boitier électronique selon l'invention comporte une plateforme inertielle 110,111,112 configurée pour générer un ensemble de données sur la démarche d'un utilisateur de la paire 10 de semelles.

Au cours de la marche d'un utilisateur, la plateforme inertielle 110 acquière des signaux représentatifs d'un paramètre de mouvement (accélération et/ou vitesse, par exemple vitesse angulaire) du pied selon les axes X, Y, Z. En outre, ces données peuvent ensuite être traitées pour générer au moins un signal d'accélération. La plateforme inertielle est par exemple constituée d'au moins un accéléromètre et un gyroscope. De façon préférée, elle comporte plusieurs accéléromètres et gyroscopes. Le boitier électronique peut également comporter un ou plusieurs magnétomètres de façon à acquérir trois signaux bruts supplémentaires correspondant aux valeurs de champs magnétiques sur trois dimensions.

Chaque boitier électronique peut comporter par ailleurs d'autres capteurs, notamment un inclinomètre, un baromètre, un capteur de température et un altimètre pour bénéficier d'une précision accrue.

En outre, le boitier électronique selon l'invention comporte un module de traitement de données 120,121,122 pouvant être configuré pour transformer l'ensemble des données générées grâce à des algorithmes prédéfinis.

Ce module de traitement, intégré au boitier électronique, peut être utilisé pour prétraiter les données générées par les capteurs de mouvement et générer les valeurs d'orientation de la semelle. Ensuite, ces données peuvent être envoyés à un terminal externe 20 comme présenté à la figure 5 pour générer les valeurs de paramètres biomécaniques avancés de la démarche.

Alternativement, comme présenté à la figure 6, le module de traitement, intégré au boitier électronique, peut être utilisé pour acquérir les données générées par les capteurs de mouvement et les envoyer à un terminal externe 20 pour générer les valeurs de paramètres biomécaniques avancés de la démarche.

Ainsi, le boitier électronique selon l'invention comporte un module de stockage 130,131,132 de données, configuré pour mémoriser au moins une partie des données transformées et/ou des données générées par le module de traitement. Le système selon l'invention est tel qu'il permet de fonctionner avec un module de stockage de données de faible capacité. Il peut être configuré pour mémoriser les données générées par la plateforme inertielle. Avantageusement, le module de stockage 130,131,132 de données est configuré pour mémoriser au moins une partie des données transformées mais ne pas mémoriser les données générées. Ainsi, sa capacité n'est pas grevée par des données brutes générées. Les données transformées peuvent correspondre à des données prétraitées par le module de traitement ou à des paramètres biomécaniques.

En outre, le boitier électronique selon l'invention comporte des moyens de communications. Ainsi, en particulier, chacun des boîtiers, qu'il soit Slave ou Master, est conçu de manière à pouvoir communiquer indépendamment avec l'autre et/ou directement avec le terminal afin de pouvoir échanger ses propres informations sur la posture/le mouvement/l'activité de son pied, dont il a reçu les données via les différents capteurs de sa semelle intérieure et/ou extérieure de chaussure

De préférence, le boitier électronique selon l'invention comporte un premier moyen de communication 140,141,142 configuré de façon à ce que le boitier électronique 100 d'au moins une des semelles soit apte à transmettre au moins une partie des données à un terminal externe 20. Ces données peuvent être transmises en temps réel ou en différé à un terminal externe 20. Le terminal externe 20 peut par exemple être un système distant tel qu'une tablette, un téléphone mobile (« smartphone » en terminologie anglosaxonne), un ordinateur ou un serveur.

Avantageusement, chaque boitier électronique comporte en outre un second moyen de communication configuré de façon à ce que le boitier électronique 101 d'une première semelle soit apte à communiquer avec le boitier électronique 102 d'une seconde semelle. En particulier, les deux boitiers électroniques sont configurés pour communiquer entre eux et pour initier la génération de données sur le mouvement du pied d'un utilisateur seulement après réception d'un message de l'autre boitiers électronique.

Les premiers et deuxièmes moyens de communication peuvent consister en un seul et même moyen.

Les premiers et deuxièmes moyens de communication sont aptes à recevoir et à transmettre les données sur au moins un réseau de communication. De préférence la communication est opérée par l'intermédiaire d'un protocole sans fils tel que wifi, 3G, 4G, et/ou Bluetooth. De préférence le protocole de communication est un protocole BLE ou ANT+. Ces protocoles de communications permettent une consommation faible en énergie.

Avantageusement, du fait de son confinement à l'intérieur d'un boitier placé sous le corps d'une personne, l'antenne doit de préférence être disposée à l'intérieur du boitier sur le côté orienté vers le côté extérieur de la semelle. Ce positionnement de l'antenne est préférable dans la mesure où des tests en laboratoire ont permis d'établir que le signal émis depuis une semelle ou une chaussure est absorbée à 70 % par le corps humain. Cette antenne doit donc être positionnée en périphérie du pied et orientée de telle manière à toujours pouvoir transmettre le signal au terminal externe et/ou au boitier de la seconde semelle. De façon préférée, l'antenne peut être une antenne imprimée sur une carte électronique. Alternativement, l'antenne peut être imprimée sur une face intérieure du boitier et connectée à la carte électronique par câblage. L'antenne peut de préférence être positionnée sur une partie basse par rapport à la carte électronique. Ainsi, la carte électronique vient faire contact avec l'antenne.

En outre, le boitier électronique selon l'invention comporte une source d'énergie 160,161,162. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

En outre, le boitier électronique selon l'invention peut comporter un moyen de connexion filaire 160, de préférence protégé par une languette amovible. Ce moyen de connexion filaire peut être par exemple un port USB ou firewire. Avantageusement, le port USB est également résistant à l'eau ou l'humidité. En outre, le port USB est avantageusement surmonté d'une poutrelle en polymère permettant de lui conférer une plus grande résistance en condition d'utilisation. Ce moyen de connexion filaire peut être utilisé comme évoqué ci-dessus pour recharger la batterie mais également pour échanger des données et par exemple mettre à jour le micrologiciel de la carte électronique portant les différents composants du boitier électronique.

De préférence, la languette amovible ou USB cover, permet de protéger le port USB de corps étrangers. Par exemple, la languette amovible permet de protéger le port USB de l'eau ou de la poussière. Une telle languette peut de préférence être réalisée en polymère de type élastomère ou polyuréthane.

Ces différents composants du boitier électronique sont de préférence agencés sur une carte électronique 170 (ou circuit imprimé). En outre, les différents moyens et modules du boitier électronique sont représentés de façon distincte sur les figures 2 et 3 mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique. En outre, lorsque l'on prête une action à un dispositif, un moyen ou un module, celle-ci est en fait effectuée par un microprocesseur du dispositif ou module commandé par des codes instructions enregistrés dans une mémoire. De même, si l'on prête une action à une application, celle-ci est en fait effectuée par un microprocesseur du dispositif dans une mémoire duquel les codes instructions correspondant à l'application sont enregistrés. Lorsqu'un dispositif ou module émet ou reçoit un message, ce message est émis ou reçu par une interface de communication.

En outre, le système 1 comporte un terminal externe 20 apte à recevoir des données. Le terminal externe 20 est généralement une tablette, un téléphone mobile (« smartphone » en terminologie anglosaxonne), une passerelle, un routeur, un ordinateur ou un serveur. Il peut être apte à transférer ces données à un serveur distant. Il est alors par exemple possible d'accéder à ce serveur distant via une interface web.

Ainsi, l'utilisateur peut accéder à des données liées à ses activités physiques quotidiennes et relatives à plusieurs paramètres biomécaniques, comme la posture, la pronation/supination, la force d'impact, la longueur des pas, le temps de contact, la boiterie, l'équilibre et plusieurs autres paramètres relatifs à l'utilisateur et décrivant ses déplacements, sa marche, ses postures et ses mouvements et ainsi de suivre leur évolution.

Avantageusement, une application dédiée est installée sur ce terminal externe afin de traiter les informations transmises par les boitiers et permettre à l'utilisateur d'interagir avec l'invention. Il est alors par exemple possible d'accéder à ce serveur distant via une interface web. Toutes les communications avec le serveur distant peuvent être sécurisées par exemple par des protocoles HTTPS et cryptage AES 512. Ainsi, cela peut permettre, via un client, l'accès aux données par du personnel médical en charge du suivi de l'utilisateur.

En outre, le boitier électronique selon l'invention comporte une **source d'énergie 160,161,162.** La source d'énergie est de préférence de type batterie, rechargeable ou non. De préférence la source d'énergie est une batterie rechargeable. La recharge peut être réalisée selon différentes technologies telles que :

- par chargeur, avec un connecteur affleurant au niveau de la semelle ;
- avec un dispositif de recharge mécanique intégré à la semelle, comme par exemple un dispositif piézoélectrique apte à fournir une énergie électrique à partir de la marche ;
- avec un dispositif sans contact, par exemple par induction ; et/ou
- avec un dispositif photovoltaïque.

En outre, le boitier électronique selon l'invention peut comporter un moyen de connexion filaire 180, de préférence protégé par une languette amovible. Ce moyen de connexion filaire peut être par exemple un port USB ou firewire. Ce moyen de connexion filaire peut être utilisé comme évoqué ci-dessus pour recharger la batterie mais également pour échanger des données et par exemple mettre à jour le micrologiciel de la carte électronique portant les différents composants du boitier électronique.

Avantageusement, le système 1 est configuré pour mettre en oeuvre les valeurs de paramètres biomécaniques dans un ou plusieurs algorithmes, de préférence préalablement calibrés. Ces algorithmes peuvent avoir été construits à partir de différents modèles d'apprentissage, notamment de partitionnement, supervisés ou non supervisés. Un algorithme d'apprentissage non supervisé peut par exemple être sélectionné parmi un modèle de mélange Gaussien non supervisé, une classification ascendante hiérarchique (Hierarchical clustering Agglomerative en terminologie Anglo-Saxonne), une classification descendante hiérarchique (Hierarchical clustering divisive en terminologie Anglo-Saxonne). Alternativement, l'algorithme repose sur un modèle d'apprentissage statistique supervisé configuré de façon à minimiser un risque de la règle d'ordonnancement et ainsi permettant d'obtenir des règles de prédiction plus performantes. Dans ce cas, les étapes de calcul de détermination et d'estimations peuvent être basées sur un modèle, entrainé sur un jeu de données et configuré pour prédire une étiquette (e.g. démarche similaire ou non à la démarche enregistrée). Par exemple, aux fins de la calibration, il est possible d'utiliser un jeu de données représentatif d'une situation dont l'étiquette est connue, par exemple des paramètres biomécaniques caractéristiques de la maladie de Parkinson. Le jeu de donnée peut également comprendre des étiquettes multiples. L'algorithme peut être issu de l'utilisation d'un modèle d'apprentissage statistique supervisé sélectionné par exemple parmi les méthodes à noyau (e.g. Séparateurs à Vaste Marge - Support Vector Machines SVM, Kernel Ridge Regression), les méthodes d'ensembles (e.g. Forêts aléatoires) partitionnement hiérarchique, partitionnement en k-moyenne, arbres de décision, régression logique ou les réseaux de neurones.

En outre, le terminal externe 20 peut comporter :

- un module d'alerte apte à alerter l'utilisateur, et éventuellement un personnel médical, d'une évolution anormale d'un ou plusieurs paramètres biomécaniques pouvant correspondre à l'apparition ou à un risque accru d'apparition d'une ou de plusieurs pathologie,
- un module de communication apte à contacter et informer directement un professionnel de la santé ou toute autre personne choisie et indiquée auparavant par l'utilisateur dans l'application et/ou apte à fournir régulièrement à l'utilisateur des informations liées à son activité quotidienne et l'évolution des paramètres biomécaniques dans le temps,
- un module correctif apte à proposer, par exemple via le terminal externe, des solutions pour rectifier ou prévenir la malformation en proposant des exercices physique ou des inserts correctifs à intégrer sous le pied,
- un module de pronostic apte à mesurer l'effet, et possiblement l'efficacité, d'un protocole de traitement neurologique, médical ou autre en suivant l'évolution de tout ou partie des paramètres de la marche via par exemple la valeur représentative de l'évolution de la démarche de l'utilisateur et apte à communiquer à l'utilisateur ou directement à son médecin ou toute autre personne prédéterminée tout ou partie des évolutions constatées des données recueillies de la démarche dudit utilisateur.

## Revendications

**1.** Procédé de détermination d'une valeur de paramètre biomécanique avancé de démarche à partir de données générées par un ou plusieurs capteurs de mouvement d'une semelle, ledit procédé, exécuté par un ou plusieurs modules d'analyse, comportant :

- Une étape (200) d'acquisition de données générées par un ou plusieurs capteurs de mouvement d'une semelle, lesdites données comportant des valeurs d'accélération, de vitesse angulaire et/ou de champs magnétique ;

- Une étape (300) de calcul d'une valeur d'orientation de la semelle, ladite valeur d'orientation de la semelle étant calculée par rapport à un référentiel terrestre à partir de données comportant les valeurs d'accélération, de vitesse angulaire et/ou de champs magnétique ;

- Une étape (600) d'identification d'une activité associée à une unité de mouvement, ladite identification d'une activité comportant la comparaison de valeurs de référence d'activité à des valeurs sélectionnées parmi : les valeurs d'accélération, de vitesse angulaire et/ou d'orientation de la semelle ou des valeurs de paramètres descriptifs globaux calculées à partir des valeurs d'accélération, de vitesse angulaire et/ou d'orientation de la semelle ;

- Une étape (700) d'identification d'au moins deux moments clés de l'unité de mouvement pour laquelle une activité a été identifiée, ladite étape d'identification de moment clés comportant la comparaison de valeurs de paramètres de mouvement à au moins un seuil prédéterminé, ledit seuil prédéterminé étant associé à l'activité identifiée et lesdites valeurs de paramètres de mouvement comportant les valeurs d'accélération, de vitesse angulaire et/ou d'orientation de la semelle ; et

- Une étape (800) de détermination d'une valeur de paramètre biomécanique avancé de marche, ladite détermination comportant le calcul d'une valeur de paramètre biomécanique de démarche pour au moins un des moments clés identifiés et à partir des valeurs d'accélération, de vitesse angulaire et/ou d'orientation de la semelle.

**2.** Procédé selon la revendication 1 **caractérisé en ce que** le paramètre biomécanique de démarche est sélectionné parmi : vitesse de propulsion, taux de fatigue, angle de Fick, direction de propulsion et direction de décélération.

**3.** Procédé selon l'une des revendications 1 ou 2, **caractérisé en ce que** les moments clés comportent : une pose du talon, une pose des orteils, une levée du talon et/ou un décollage des orteils.

**4.** Procédé selon l'une quelconque des revendications 1 à 3, **caractérisé en ce qu'**il comporte en outre une étape (400) de prétraitement des valeurs d'accélération, de vitesse angulaire et d'orientation de la semelle, ladite étape (400) de prétraitement comportant au moins un traitement sélectionné parmi : un filtrage fréquentiel, une suppression de la gravité sur les valeurs d'accélération, une suppression de la gravité, une suppression du bruit et/ou de la dérive sur les valeurs d'accélération, de vitesse angulaire et d'orientation de la semelle.

**5.** Procédé selon la revendication 4 **caractérisé en ce que** l'étape (400) de prétraitement est réalisée dans un boitier électronique intégré à la semelle.

**6.** Procédé selon l'une quelconque des revendications 1 à 5, **caractérisé en ce qu'**il comporte en outre une étape (500) d'identification d'une unité de mouvement.

**7.** Procédé selon l'une quelconque des revendications 1 à 6, **caractérisé en ce que** l'étape (600) d'identification d'une activité associée à une unité de mouvement comporte le calcul d'un ou plusieurs paramètres descriptifs globaux à partir des valeurs d'accélération, de vitesse angulaire et/ou de champs magnétique, lesdits paramètres descriptifs globaux comportant une valeur de kurtosis ou de coefficient d'asymétrie.

**8.** Procédé selon l'une quelconque des revendications 1 à 6, **caractérisé en ce que** l'étape (600) d'identification d'une activité associée à une unité de mouvement comporte le calcul d'un ou plusieurs paramètres descriptifs globaux à partir des valeurs d'accélération, de vitesse angulaire et/ou de champs magnétique, lesdits paramètres descriptifs globaux comportant une ou plusieurs valeurs de coefficients de similarité avec des motifs prédéterminés.

**9.** Procédé selon l'une quelconque des revendications 1 à 8, **caractérisé en ce que** l'étape (600) d'identification d'une activité associée à une unité de mouvement comporte une étape de reconnaissance probabiliste et/ou une étape de reconnaissance déterministe.

**10.** Procédé selon l'une quelconque des revendications 1 à 9, **caractérisé en ce que** l'étape (700) d'identification d'au moins deux moments clés est réalisée dans un boitier électronique intégré à la semelle.

**11.** Procédé selon l'une quelconque des revendications 1 à 10, **caractérisé en ce que** l'étape (800) de détermination d'une valeur de paramètre biomécanique avancé de marche est réalisée dans un boitier électronique intégré à la semelle.

**12.** Procédé selon l'une quelconque des revendications 1 à 11, **caractérisé en ce qu'**il comporte en outre une étape de calcul d'un motif combinatoire de valeurs de paramètres biomécaniques avancés, ledit motif combinatoire de valeurs de paramètres biomécaniques avancés correspondant à une combinaison de valeurs de paramètres biomécaniques avancés ou à une combinaison de comportement en fonction du temps de paramètres biomécaniques avancés.

**13.** Système (1) de détermination d'une valeur de paramètre biomécanique avancé de démarche à partir de données générées par un ou plusieurs capteurs de mouvement d'une semelle, **caractérisé en ce qu'**il comporte :

- Deux ensembles d'un ou de plusieurs capteurs de mouvement d'une semelle (101, 102), un premier ensemble étant apte à être associé à une première semelle ou chaussure et un second ensemble étant apte à être associé à une seconde semelle ou chaussure, chaque ensemble étant configurés pour générer des données comportant des valeurs d'accélération, de vitesse angulaire et/ou de champs magnétique :
- Un ou plusieurs modules d'analyse (120, 121, 122, 220) configurés pour déterminer d'une valeur de paramètre biomécanique avancé de démarche, lesdits un ou plusieurs modules d'analyse étant configurés pour :

-- Acquérir les données générées par les un ou plusieurs capteurs de mouvement ;
-- Calculer une valeur d'orientation de la semelle, ladite valeur d'orientation de la semelle étant calculée par rapport à un référentiel terrestre à partir de données comportant les valeurs d'accélération, de vitesse angulaire et/ou de champs magnétique ;
-- Identifier une activité associée à une unité de mouvement, ladite identification d'une activité comportant la comparaison de valeurs de référence d'activité à des valeurs sélectionnées parmi : les valeurs d'accélération, de vitesse angulaire, et/ou d'orientation de la semelle ou des valeurs de paramètres descriptifs globaux calculées à partir des valeurs d'accélération, de vitesse angulaire et/ou d'orientation de la semelle ;
-- Identifier au moins deux moments clés de l'unité de mouvement pour laquelle une activité a été identifiée, ladite étape d'identification de moment clés comportant la comparaison de valeurs de paramètres de mouvement à au moins un seuil prédéterminé, ledit seuil prédéterminé étant associé à l'activité identifiée et lesdites valeurs de paramètres de mouvement comportant les valeurs d'accélération, de vitesse angulaire et/ou d'orientation de la semelle ; et

- Déterminer une valeur de paramètre biomécanique avancé de marche, ladite détermination comportant le calcul d'une valeur de paramètre biomécanique de démarche pour au moins un des moments clés identifiés et à partir des valeurs d'accélération, de vitesse angulaire et/ou d'orientation de la semelle.

**14.** Système (1) de détermination d'une valeur de paramètre biomécanique avancé de démarche selon la revendication 13, **caractérisé en ce que** la paire d'ensemble d'un ou de plusieurs capteurs de mouvement d'une semelle (101, 102), correspond à une paire de boitiers électroniques aptes à être intégrés dans une paire (10) de semelles.

**15.** Système (1) de détermination d'une valeur de paramètre biomécanique avancé de démarche selon la revendication 14, **caractérisé en ce qu'**il comporte plusieurs modules de traitement configurés pour chacun exécuter une partie de la détermination d'une valeur de paramètre biomécanique avancé de démarche.

**Patentansprüche**

**1.** Verfahren zur Bestimmung eines Wertes eines fortgeschrittenen biomechanischen Gangparameters aus von einem oder mehreren Bewegungssensoren einer Sohle erzeugten Daten, wobei das von einem oder mehreren Analysemodulen ausgeführte Verfahren aufweist:

- einen Schritt (200) des Erfassens von von einem oder mehreren Bewegungssensoren einer Sohle erzeugten Daten, wobei die Daten Beschleunigungs-, Winkelgeschwindigkeits- und/oder Magnetfeldwerte aufweisen;
- einen Schritt (300) des Berechnens eines Ausrichtungswertes der Sohle, wobei der Ausrichtungswert der Sohle im Verhältnis zu einem terrestrischen Bezugssystem aus Daten berechnet wird, die die Beschleunigungs-, Winkelgeschwindigkeits- und/oder Magnetfeldwerte aufweisen;
- einen Schritt (600) des Identifizierens einer Aktivität, die einer Bewegungseinheit zugeordnet ist, wobei die Identifizierung einer Aktivität den Vergleich von Aktivitätsreferenzwerten mit Werten aufweist, die ausgewählt sind aus: den Beschleunigungs-, Winkelgeschwindigkeits- und/oder Ausrichtungswerten der Sohle oder den Werten allgemein beschreibender Parameter, berechnet aus Beschleunigungs-, Winkelgeschwindigkeits- und/oder Ausrichtungswerten der Sohle;

- einen Schritt (700) des Identifizierens von mindestens zwei Schlüsselmomenten der Bewegungseinheit, für die eine Aktivität identifiziert wurde, wobei der Schritt des Identifizierens eines Schlüsselmoments den Vergleich von Bewegungsparameterwerten mit mindestens einem vorher festgelegten Grenzwert aufweist, wobei der vorher festgelegte Grenzwert der identifizierten Aktivität zugeordnet ist und die Bewegungsparameterwerte die Beschleunigungs-, Winkelgeschwindigkeits- und/oder Ausrichtungswerte der Sohle aufweisen; und

- einen Schritt (800) des Bestimmens eines Wertes eines fortgeschrittenen biomechanischen Gangparameters, wobei die Bestimmung die Berechnung eines Wertes eines biomechanischen Gangparameters für mindestens einen der identifizierten Schlüsselmomente und ausgehend von den Beschleunigungs-, Winkelgeschwindigkeits- und/oder Ausrichtungswerten der Sohle aufweist.

2. Verfahren nach Anspruch 1, **dadurch gekennzeichnet, dass** der biomechanische Gangparameter ausgewählt ist aus: Antriebsgeschwindigkeit, Ermüdungsgrad, Fick-Winkel, Antriebsrichtung und Entschleunigungsrichtung.

3. Verfahren nach einem der Ansprüche 1 oder 2, **dadurch gekennzeichnet, dass** die Schlüsselmomente aufweisen: ein Aufsetzen der Ferse, ein Aufsetzen der Zehen, ein Anheben der Ferse und/oder ein Lösen der Zehen.

4. Verfahren nach einem der Ansprüche 1 bis 3, **dadurch gekennzeichnet, dass** es ferner einen Schritt (400) des Vorverarbeitens der Beschleunigungs-, Winkelgeschwindigkeits- und Ausrichtungswerte der Sohle aufweist, wobei der Schritt (400) des Vorverarbeitens mindestens eine ausgewählte Verarbeitung aufweist aus: einer Frequenzfilterung, einer Eliminierung der Schwerkraft auf die Beschleunigungswerte, einer Eliminierung der Schwerkraft, einer Eliminierung des Geräuschs und/oder der Ablenkung auf die Beschleunigungs-, Winkelgeschwindigkeits- und Ausrichtungswerte der Sohle.

5. Verfahren nach Anspruch 4 **dadurch gekennzeichnet, dass** der Schritt (400) des Vorverarbeitens in einem in der Sohle integrierten Elektronikkasten durchgeführt wird.

6. Verfahren nach einem der Ansprüche 1 bis 5, **dadurch gekennzeichnet, dass** es ferner einen Schritt (500) des Identifizierens einer Bewegungseinheit aufweist.

7. Verfahren nach einem der Ansprüche 1 bis 6, **dadurch gekennzeichnet, dass** der Schritt (600) des Identifizierens einer Aktivität, die einer Bewegungseinheit zugeordnet ist, die Berechnung von einem oder mehreren allgemein beschreibenden Parametern ausgehend von den Beschleunigungs-, Winkelgeschwindigkeits- und/oder Magnetfeldwerten aufweist, wobei die allgemein beschreibenden Parameter einen Kurtosiswert oder Asymmetriekoeffizienten aufweisen.

8. Verfahren nach einem der Ansprüche 1 bis 6, **dadurch gekennzeichnet, dass** der Schritt (600) des Identifizierens einer Aktivität, die einer Bewegungseinheit zugeordnet ist, die Berechnung von einem oder mehreren allgemein beschreibenden Parametern ausgehend von den Beschleunigungs-, Winkelgeschwindigkeits- und/oder Magnetfeldwerten aufweist, wobei die allgemein beschreibenden Parameter einen oder mehrere Ähnlichkeitskoeffizientwerte mit vorher festgelegten Motiven aufweisen.

9. Verfahren nach einem der Ansprüche 1 bis 8, **dadurch gekennzeichnet, dass** der Schritt (600) des Identifizierens einer Aktivität, die einer Bewegungseinheit zugeordnet ist, einen Schritt des probabilistischen Wiedererkennens und/oder einen Schritt des deterministischen Wiedererkennens aufweist.

10. Verfahren nach einem der Ansprüche 1 bis 9, **dadurch gekennzeichnet, dass** der Schritt (700) des Identifizierens von mindestens zwei Schlüsselmomenten in einem in der Sohle integrierten Elektronikkasten durchgeführt wird.

11. Verfahren nach einem der Ansprüche 1 bis 10, **dadurch gekennzeichnet, dass** der Schritt (800) des Bestimmens eines Wertes eines fortgeschrittenen biomechanischen Gangparameters in einem in der Sohle integrierten Elektronikkasten durchgeführt wird.

12. Verfahren nach einem der Ansprüche 1 bis 11, **dadurch gekennzeichnet, dass** es ferner einen Schritt des Berechnens eines kombinatorischen Wertemotivs fortgeschrittener biomechanischer Parameter aufweist, wobei das kombinatorische Wertemotiv fortgeschrittener biomechanischer Parameter einer Wertekombination fortgeschrittener biomechanischer Parameter oder einer zeitabhängigen Verhaltenskombination fortgeschrittener biomechanischer Parameter entspricht.

**13.** System (1) zur Bestimmung eines Wertes eines fortgeschrittenen biomechanischen Gangparameters aus von einem oder mehreren Bewegungssensoren einer Sohle erzeugten Daten, **dadurch gekennzeichnet, dass** es aufweist:

- zwei Anordnungen aus einem oder mehreren Bewegungssensoren einer Sohle (101, 102), wobei eine erste Anordnung imstande ist, einer ersten Sohle oder Schuh zugeordnet zu sein, und eine zweite Anordnung imstande ist, einer zweiten Sohle oder Schuh zugeordnet zu sein, wobei jede Anordnung dazu ausgelegt ist, Daten zu erzeugen, die Beschleunigungs-, Winkelgeschwindigkeits- und/oder Magnetfeldwerte aufweisen:
- ein oder mehrere Analysemodule (120, 121, 122, 220), die ausgelegt sind, einen Wert eines fortgeschrittenen biomechanischen Gangparameters zu bestimmen, wobei das eine oder die mehreren Analysemodule ausgelegt sind, um:

-- die von den einen oder mehreren Bewegungssensoren erzeugten Daten zu erfassen;
-- einen Ausrichtungswert der Sohle zu berechnen, wobei der Ausrichtungswert der Sohle im Verhältnis zu einem terrestrischen Bezugssystem aus Daten berechnet wird, die die Beschleunigungs-, Winkelgeschwindigkeits- und/oder Magnetfeldwerte aufweisen;
-- eine Aktivität zu identifizieren, die einer Bewegungseinheit zugeordnet ist, wobei die Identifizierung einer Aktivität den Vergleich von Aktivitätsreferenzwerten mit Werten aufweist, die ausgewählt sind aus: den Beschleunigungs-, Winkelgeschwindigkeits- und/oder Ausrichtungswerten der Sohle oder den Werten allgemein beschreibender Parameter, berechnet aus Beschleunigungs-, Winkelgeschwindigkeits- und/oder Ausrichtungswerten der Sohle;
-- mindestens zwei Schlüsselmomente der Bewegungseinheit zu identifizieren, für die eine Aktivität identifiziert wurde, wobei der Schritt des Identifizierens von Schlüsselmomenten den Vergleich von Bewegungsparameterwerten mit mindestens einem vorher festgelegten Grenzwert aufweist, wobei der vorher festgelegte Grenzwert der identifizierten Aktivität zugeordnet ist und die Bewegungsparameterwerte die Beschleunigungs-, Winkelgeschwindigkeits- und/oder Ausrichtungswerte der Sohle umfassen; und

- einen Wert eines fortgeschrittenen biomechanischen Gangparameters zu bestimmen, wobei die Bestimmung die Berechnung eines biomechanische Gangparameterwertes für mindestens einen der identifizierten Schlüsselmomente und ausgehend von den Beschleunigungs-, Winkelgeschwindigkeits- und/oder Ausrichtungswerten der Sohle aufweist.

**14.** System (1) zur Bestimmung eines Wertes eines fortgeschrittenen biomechanischen Gangparameters nach Anspruch 13, **dadurch gekennzeichnet, dass** das Anordnungspaar aus einem oder mehreren Bewegungssensoren einer Sohle (101, 102) einem Paar von Elektronikgehäusen entspricht, die imstande sind, in ein Paar (10) von Sohlen integriert zu sein.

**15.** System (1) zur Bestimmung eines Wertes eines fortgeschrittenen biomechanischen Gangparameters nach Anspruch 14, **dadurch gekennzeichnet, dass** es mehrere Verarbeitungsmodule aufweist, die dazu ausgelegt sind, dass jedes einen Teil der Bestimmung eines Wertes eines fortgeschrittenen biomechanischen Gangparameters ausführt.

**Claims**

**1.** A method for determining a value of an advanced biomechanical gait parameter on the basis of data generated by one or more motion sensors in a sole, said method, executed by one or more analysis modules, comprising:

- a step (200) of acquiring data generated by one or more motion sensors in a sole, said data comprising values for acceleration, angular velocity and/or magnetic field;
- a step (300) of calculating a value for the orientation of the sole, said value for the orientation of the sole being calculated with respect to a terrestrial reference frame on the basis of data comprising values for acceleration, angular velocity and/or magnetic field;
- a step (600) of identifying an activity associated with a unit of movement, said identification of an activity comprising the comparison of reference activity values with selected values from: values for acceleration, angular velocity and/or orientation of the sole, or values of global descriptive parameters calculated on the basis of the values for acceleration, angular velocity and/or orientation of the sole;
- a step (700) of identifying at least two key moments in the unit of movement for which an activity has been identified, said step of identifying key moments comprising the comparison of values of movement parameters with at least one predetermined threshold, said predetermined threshold being associated with the identified activity and said values of movement parameters comprising values for acceleration, angular velocity and/or orientation of sole; and
- a step (800) of determining a value of an advanced biomechanical gait parameter, said determining operation comprising the calculation of a value of a biomechanical gait parameter for at least one of the key moments identified and on the basis of values for acceleration, angular velocity and/or orientation of the sole.

2. The method according to claim 1 **characterised in that** the biomechanical gait parameter is selected from: speed of propulsion, rate of fatigue, Fick angle, direction of propulsion and direction of deceleration.

3. The method according to one of claims 1 or 2, **characterised in that** the key moments comprise: a heel strike, toe strike, heel lift and/or toe off.

4. The method according to any one of claims 1 to 3, **characterised in that** it further comprises a step (400) of pre-processing of the values for acceleration, angular velocity and orientation of the sole, said pre-processing step (400) comprising at least one process selected from: frequency filtering, gravity suppression on the acceleration values, gravity suppression, noise suppression and/or drift suppression on the values for acceleration, angular velocity and orientation of the sole.

5. The method according to claim 4 **characterised in that** the pre-processing step (400) is performed in an electronic unit incorporated in the sole.

6. The method according to any one of claims 1 to 5, **characterised in that** it further comprises a step (500) of identifying a unit of movement.

7. The method according to any one of claims 1 to 6, **characterised in that** the step (600) of identifying an activity associated with a unit of movement comprises calculating one or more global descriptive parameters on the basis of values for acceleration, angular velocity and/or magnetic field, said global descriptive parameters comprising a kurtosis value or asymmetry coefficient.

8. The method according to any one of claims 1 to 6, **characterised in that** the step (600) of identifying an activity associated with a unit of movement comprises calculating one or more global descriptive parameters on the basis of values for acceleration, angular velocity and/or magnetic field, said global descriptive parameters comprising one or more values for coefficients of similarity with predetermined patterns.

9. The method according to any one of claims 1 to 8, **characterised in that** the step (600) of identifying an activity associated with a unit of movement comprises a probabilistic recognition step and/or a deterministic recognition step.

10. The method according to any one of claims 1 to 9, **characterised in that** the step (700) of identifying at least two key moments is carried out in an electronic unit incorporated in the sole.

11. The method according to any one of claims 1 to 10, **characterised in that** the step (800) of determining a value of an advanced biomechanical gait parameter is carried out in an electronic unit incorporated in the sole.

12. The method according to any one of claims 1 to 11, **characterised in that** it further comprises a step of calculating a combinatorial pattern of values of advanced biomechanical parameters, said combinatorial pattern of values of advanced biomechanical parameters corresponding to a combination of values of advanced biomechanical parameters or a combination of behaviour as a function of time of advanced biomechanical parameters.

13. A system (1) for determining a value of an advanced biomechanical gait parameter on the basis of data generated by one or more motion sensors of a sole, **characterised in that** it comprises:

- two assemblies of one or more motion sensors of a sole (101, 102), a first assembly being able to be combined with a first sole or shoe and a second assembly being able to be combined with a second sole or shoe, each assembly being configured to generate data comprising values for acceleration, angular velocity and/or magnetic field:
- one or more analysis modules (120, 121, 122, 220) configured to determine a value of an advanced biomechanical gait parameter, said one of more analysis modules being configured for:

-- acquiring the data generated by the one or more motion sensors;
-- calculating a value of the orientation of the sole, said value of the orientation of the sole being calculated with respect to a terrestrial reference frame on the basis of data comprising values for acceleration, angular velocity and/or magnetic field;
-- identifying activity associated with a unit of movement, said identification of an activity comprising the comparison of reference activity values with values selected from: the values for acceleration, angular velocity,

and/or orientation of the sole or values of global descriptive parameters calculated on the basis of values for acceleration, angular velocity and/or orientation of the sole;

-- identifying at least two key moments in the unit of movement for which an activity has been identified, said step of identifying key moments comprising the comparison of values of movement parameters with at least one predetermined threshold, said predetermined threshold being associated with the identified activity and said values of movement parameters comprising the values for acceleration, angular velocity and/or orientation of sole and

- determining a value of an advanced biomechanical gait parameter, said determining operation comprising the calculation of a value of a biomechanical gait parameter for at least one of the key moments identified and on the basis of values for acceleration, angular velocity and/or orientation of the sole.

**14.** The system (1) for determining a value of an advanced biomechanical gait parameter according to claim 13, **characterised in that** the assembly pair of one or more motion sensors of a sole (101, 102) corresponds to a pair of electronic units capable of being incorporated in a pair (10) of soles.

**15.** The system (1) for determining a value of an advanced biomechanical gait parameter according to claim 14, **characterised in that** it comprises a plurality of processing modules configured to each execute a part of the determination of a value of an advanced biomechanical gait parameter.

100 — ┌ - - - - - - - - - - - - - - - - - - - - - - - - - - - - - - - - - ┐
     │                          Apprentissage                          │
     └ - - - - - - - - - - - - - - - - - - - - - - - - - - - - - - - - - ┘

200 — ┌─────────────────────────────────────────────────────────────────┐
     │ Acquisition des données générées comportant des valeurs          │
     │ d'accélération, de vitesse angulaire et/ou de champs magnétique   │
     └─────────────────────────────────────────────────────────────────┘

210 — ┌ - - - - - - - - - - - - - - - - - - - - - - - - - - - - - ┐
     │                        Calibration                        │
     └ - - - - - - - - - - - - - - - - - - - - - - - - - - - - - ┘

300 — ┌─────────────────────────────────────────────────────────────────┐
     │ Calcul d'une valeur d'orientation de la semelle par rapport à un  │
     │ référentiel terrestre à partir des données générées              │
     └─────────────────────────────────────────────────────────────────┘

400 — ┌ - - - - - - - - - - - - - - - - - - - - - - - - - - - - - - - - - ┐
     │ Prétraitement des valeurs d'accélération, de vitesse angulaire    │
     │ et/ou de champs magnétique                                        │
     └ - - - - - - - - - - - - - - - - - - - - - - - - - - - - - - - - - ┘

500 — ┌ - - - - - - - - - - - - - - - - - - - - - - - - - - - - - - - - - ┐
     │              Identification d'une unité de mouvement              │
     └ - - - - - - - - - - - - - - - - - - - - - - - - - - - - - - - - - ┘

600 — ┌─────────────────────────────────────────────────────────────────┐
     │ Identification d'une activité associée à une unité de mouvement   │
     └─────────────────────────────────────────────────────────────────┘

700 — ┌─────────────────────────────────────────────────────────────────┐
     │ Identification d'au moins deux moments clés de l'unité de         │
     │ mouvement pour laquelle une activité a été identifiée             │
     └─────────────────────────────────────────────────────────────────┘

800 — ┌─────────────────────────────────────────────────────────────────┐
     │ Détermination d'une valeur de paramètre biomécanique avancé       │
     │ de marche pour au moins un moment clés identifié                  │
     └─────────────────────────────────────────────────────────────────┘

900 — ┌ - - - - - - - - - - - - - - - - - - - - - - - - - - - - - - - - - ┐
     │ Transmission de l'ensemble des données générées et/ou            │
     │ calculées à un terminal externe                                   │
     └ - - - - - - - - - - - - - - - - - - - - - - - - - - - - - - - - - ┘

950 — ┌ - - - - - - - - - - - - - - - - - - - - - - - - - - - - - - - - - ┐
     │ Mémorisation de l'ensemble des données reçues, générées et/ou    │
     │ calculées par un terminal externe                                 │
     └ - - - - - - - - - - - - - - - - - - - - - - - - - - - - - - - - - ┘

**FIG. 1**

**FIG. 2**

**FIG. 3**

EP 3 958 738 B1

**FIG. 4**

**FIG. 5**

25

**EP 3 958 738 B1**

## RÉFÉRENCES CITÉES DANS LA DESCRIPTION

**Documents brevets cités dans la description**

- EP 3038042 A1

**Littérature non-brevet citée dans la description**

- **REBULA et al.** *Gait Posture.,* Septembre 2013, vol. 38 (4), 974-980
- **MARIANI et al.** *IEEE Trans. Bio-Med. Eng.,* 2012, vol. 59, 3162-3168
- **BENOUSSAAD et al.** *Sensors (Basel).,* 23 Décembre 2015, vol. 16 (1), E12